# EUROPEAN PATENT APPLICATION

(11) **EP 2 599 431 A1**
(43) Date of publication of application: **05.06.2013**
(21) Application number: 12782852.3
(22) Date of filing: 26.04.2012
(51) Int. Cl.: A61B 1/00

(54) **MEDICAL CONTROL DEVICE**

(30) Priority: 12.05.2011 JP 2011107424; 12.05.2011 JP 2011107423
(71) Applicant: Olympus Medical Systems Corp., Tokyo 151-0072 (JP)
(72) Inventor: UMEMOTO Yoshitaka, Tokyo 151-0072 (JP)
(74) Representative: Gunzelmann, Rainer
(86) International application number: PCT/JP2012/061202
(87) International publication number: WO 2012/153646

(57) **Abstract**

A medical control apparatus is provided in a medical apparatus and includes: an insertion portion that is provided in a medical apparatus and comprises a bending portion on a distal end side; a drive portion that generates a driving force for subjecting the bending portion to a bending operation; a wire that is connected with slackness to the bending portion; a pulling portion that pulls the wire; a connecting portion that connects the drive portion and the pulling portion so as to have a positional relationship that transmits/does not transmit a driving force between the drive portion and the pulling portion; a driving amount detection portion that detects a driving amount of the drive portion as a drive portion driving amount; a pulling portion driving amount detection portion that detects a pulling portion driving amount produced by the pulling portion; a comparison portion that compares a change amount of the drive portion driving amount and a change amount of the pulling portion driving amount; and an identification portion that, based on a comparison result obtained by the comparison portion, identifies a boundary between a range in which contribution to bending of the bending portion is possible by a restoring force of an elastic member forming the bending portion and a range in which the contribution to bending of the bending portion is not possible due to occurrence of the slackness of the wire.

## Description

### Technical Field

The present invention relates to a medical control apparatus that drives to bend a bending portion provided on a distal end side of an insertion portion by utilizing the pulling of a wire.

### Background Art

Various medical apparatuses that include a bendable bending portion have been developed in recent years. For example, an endoscope or a treatment instrument that includes a bending portion on a distal end side of an insertion portion that is inserted into a body is widely used in a medical field.

Further, treatment of an in-vivo lesion region or the like is performed using a treatment instrument that is inserted through a treatment instrument channel provided in an endoscope. Note that treatment may also be performed using a treatment instrument under endoscopic observation, without using a treatment instrument channel.

In addition, active treatment instruments and the like are in practical use that include drive means or a drive portion such as an actuator for electrically driving a bending portion to improve operability.

In a medical apparatus such as an active treatment instrument or an endoscope in which a bending portion is provided on a distal end side thereof, a configuration is adopted in which the bending portion and a drive portion are connected via an angle wire (hereunder, abbreviated as "wire"), and control is performed so as to drive the bending portion on the distal end side by pulling and driving the wire by means of the drive portion provided on a user's hand side.

In a configuration that performs control in this manner, since, for structural reasons, slackness arises in a wire that is inserted through the inside of a flexible and elongated shaft portion between the bending portion and the drive portion on the user's hand side so as to enable the wire to be inserted in a bent state into a body cavity, it is difficult to completely avoid the occurrence of a (bending) unresponsive state in which the bending portion does not bend even when the drive portion pulls the wire due to slackness of the wire.

Further, a deviation arises in the relationship between a driving amount on the drive portion side and a bending amount on the bending portion side due to slackness of the wire, and hysteresis characteristics are exhibited in which the relationship depends on past operation states.

In Japanese Patent Application Laid-Open Publication No. 6-22904 as a first conventional example that deals with slackness of a wire, an apparatus that drives to bend a bending portion is disclosed in which a rear end of a wire whose distal end is fixed to a bending portion is wound around a pulley disposed inside a connector, and a gear that is connected to a rotation shaft of the pulley and a gear that is connected to a rotation shaft of a motor as a drive portion are intermeshed.

In the aforementioned apparatus, in an endoscope operation portion that is a midway position of a wire connecting the bending portion on a distal end side of an insertion portion and the pulley that is provided inside the connector at a proximal end of a universal cord, a sensor is provided which is brought in contact with an idler whose rotational amount changes according to a movement amount of the wire and which detects the rotational amount of the idler, and a slackened state of the wire in which a driving force generated by the motor as a drive portion does not contribute to bending of the bending portion is detected by comparing a rotational amount of the motor and a rotational amount of the idler.

Further, when slackness is detected, the motor is rotated at high speed to instantly eliminate the slackened state. Further, it is disclosed that, after the slackened state has been eliminated, the bending portion is caused to bend by normal rotation of the motor.

In addition, with respect to this apparatus, a configuration is also disclosed in which, instead of an idler, a pulley is disposed inside the endoscope operation portion, and a sensor is provided that detects rotation of the pulley.

Further, in Japanese Patent Application Laid-Open Publication No. 2004-41538 as a second conventional example, a configuration is disclosed in which, when driving to bend a bending portion by means of a motor via a wire, a control apparatus drives the motor through a motor control machine based on a control signal Rmot that is outputted from a correction table.

The above described correction table of the aforementioned apparatus is configured so that, in a range of a target value R that includes an initial state (neutral reference position) in which the bending portion does not bend in the upward direction or downward direction and slackness arises in the wire, the correction table outputs the control signal Rmot so that a change amount relative to the target value R increases more than in another range, to thereby cause the motor to rotate quickly and promptly remove slackness from the wire.

However, in the first conventional example, since any countermeasure against the hysteresis characteristics due to the slackness of the wire is not taken, when the driving for bending is repeatedly performed, the relationship between the driving amount of the drive portion side and the actual bending amount of the bending portion is deviated so that precision of a control system for performing the driving for bending is lowered.

Further, the second conventional example assumes that the slackness of the wire occurs in a predetermined range including a neutral reference potion where the bending portion is not bent, but the slackness actually exerts influences when the bending portion is bent in an opposite direction even in a bent state at a position displaced from the neutral reference position. Thus, the second conventional example has a defect that it can not be applied to the bent state deviated from vicinity of the neutral reference position.

Also in the second conventional example, since any countermeasure against the hysteresis characteristics due to the slackness of the wire is not taken, likewise as explained with respect to the first conventional example, there is a defect that precision of the control system for performing the driving for bending is lowered when the driving for bending is repeatedly performed.

Further, the insertion portion including the bending portion is formed by an elastic member that is a flexible member and has elasticity. Therefore, when the bending portion is bent in one direction and then it is attempted to bend the bending portion in the opposite direction so as to decrease a bending angle, there is a case where the bending portion is bent in the opposite direction by a restoring force of the elastic member and the restoring force acts to cancel at least a part of the slackness.

The first and second conventional examples do not deal with the case where the restoring force is generated. Therefore, in the case of using the wire with slackness, in particular, if it is configured that a boundary where the bending by the restoring force stops and the slackness in the wire substantially arises is identified, it is possible to perform control to deal with the occurrence of the slackness in the circumstance where the restoring force acts, and it is made easy to deal with the driving for bending which shows the hysteresis characteristics.

In other words, it has been desired to enable identification of a boundary between a range in which contribution to the bending of the bending portion is possible by a restoring force of the elastic member forming the bending portion and a range in which the contribution to the bending of the bending portion is not possible due to occurrence of slackness of the wire.

The present invention has been made in view of the above circumstances and an object of the present invention is to provide a medical control apparatus capable of identifying a boundary between a range in which contribution to bending of a bending portion is possible by a restoring force of an elastic member forming a bending portion and a range in which the contribution to the bending of the bending portion is not possible due to occurrence of slackness of a wire.

### Disclosure of Invention

### Means for Solving the Problem

A medical control apparatus according to one aspect of the present invention includes: an insertion portion that is provided in a medical apparatus, and that comprises a bending portion that is formed using an elastic member having flexibility on a distal end side; a drive portion that generates a driving force for subjecting the bending portion to a bending operation; a wire that is inserted through an inside of the insertion portion, and provided with slackness and connected to the bending portion; a pulling portion that pulls the wire; a connecting portion that connects the drive portion and the pulling portion so as to have a positional relationship that transmits the driving force between the drive portion and the pulling portion and a positional relationship that cannot transmit the driving force between the drive portion and the pulling portion; a driving amount detection portion that detects a driving amount of the drive portion as a drive portion driving amount; a pulling portion driving amount detection portion that detects a pulling portion driving amount that is pulled by the pulling portion; a comparison portion that compares a change amount of the drive portion driving amount and a change amount of the pulling portion driving amount; and an identification portion that, based on a comparison result obtained by the comparison portion, identifies a boundary between a range in which contribution to bending of the bending portion is possible by a restoring force of the elastic member forming the bending portion and a range in which the contribution to bending of the bending portion is not possible due to occurrence of the slackness of the wire.

A medical control apparatus according to another aspect of the present invention includes: an insertion portion having a bending portion on a distal end side; a drive portion that generates a driving force for subjecting the bending portion to a bending operation; a wire that extends from the bending portion and can be pulled by means of the drive portion, and that is connected with slackness to the bending portion; a judgment portion that judges whether or not a driving force of the drive portion is in a driving force contributing state that contributes to driving to bend the bending portion; a driving force detection portion that detects a driving force generated at the drive portion; a position identification portion that detects a specific bending state position at which the bending portion maintains a specific bending state without a driving force that drives to bend the bending portion being applied by the drive portion; and a discrimination portion that discriminates, as bending states of the bending portion, a first bending state that is the driving force contributing state, a second bending state in which a bending state of the bending portion is changed as far as the specific bending state position immediately after the state is changed from the driving force contributing state to a bending state that is not the driving force contributing state by the drive portion, and a third bending state in which a driving force generated by the drive portion is not detected as a value that is greater than or equal to a predetermined value in a bending state that is not the driving force contributing state excluding the second bending state.

### Brief Description of the Drawings

Fig. 1A is a block diagram showing a configuration of a medical control apparatus according to a first embodiment of the present invention.
Fig. 1B is a block diagram showing a configuration of a comparison portion.
Fig. 1C is a block diagram showing a configuration of a modification of the comparison portion.
Fig. 1D is a block diagram showing a configuration of a discrimination portion.
Fig. 2 is a block diagram showing a configuration of a medical apparatus that includes the first embodiment.
Fig. 3 is a view showing a schematic configuration of a treatment instrument.
Fig. 4 is a view showing a motor constituting a drive portion of a treatment instrument, a structure of a portion involved in bending that includes a connecting portion with a pulley to which a rear end of a wire is connected, and a simplified model of the structure.
Fig. 5 is a view that illustrates representative bending states in a case where the motor is rotated to drive to bend the bending portion using the model shown in Fig. 4, and corresponding motor rotation angles, pulley rotation angles, and torques of the motor.
Fig. 6A is an explanatory view of a case where a bending angle of the bending portion and a motor rotation angle exhibit a hysteresis characteristic that corresponds to Fig. 5.
Fig. 6B is an explanatory view of a case where a motor rotation angle and a pulley bending angle exhibit a hysteresis characteristic that corresponds to Fig. 5.
Fig. 7 is a flowchart illustrating a representative example of control procedures performed by a control apparatus in the first embodiment.
Fig. 8 is a flowchart illustrating details with respect to some steps in the flowchart shown in Fig. 7.
Fig. 9A is a block diagram showing a configuration of a medical control apparatus according to a second embodiment of the present invention.
Fig. 9B is a block diagram showing a configuration example of a comparison portion.
Fig. 10 is a block diagram showing a modification of the comparison portion.
Fig. 11 is a block diagram showing a configuration of a discrimination portion.
Fig. 12 is a block diagram showing a configuration of a medical apparatus that includes the second embodiment.
Fig. 13 is a view showing a schematic configuration of a treatment instrument.
Fig. 14 is a view showing a motor constituting a drive portion of a treatment instrument, a structure of a portion involved in bending that includes a connecting portion with a pulley to which a rear end of a wire is connected, and a simplified model of the structure.
Fig. 15 is a view that illustrates representative bending states in a case where the motor is rotated to drive to bend the bending portion using the model shown in Fig. 14, and corresponding motor rotation angles, pulley rotation angles, and torques of the motor.
Fig. 16A is an explanatory view of a case where a bending angle of the bending portion and a motor rotation angle exhibit a hysteresis characteristic that corresponds to Fig. 15.
Fig. 16B is an explanatory view of a case where a motor rotation angle and a pulley bending angle exhibit a hysteresis characteristic that corresponds to Fig. 15.
Fig. 17 is a flowchart illustrating a representative example of control procedures performed by a control apparatus in the second embodiment.
Fig. 18 is a flowchart illustrating details with respect to some steps in the flowchart shown in Fig. 17.

### Best Mode for Carrying Out the Invention

Hereunder, embodiments of the present invention are described with reference to the drawings.

### (First Embodiment)

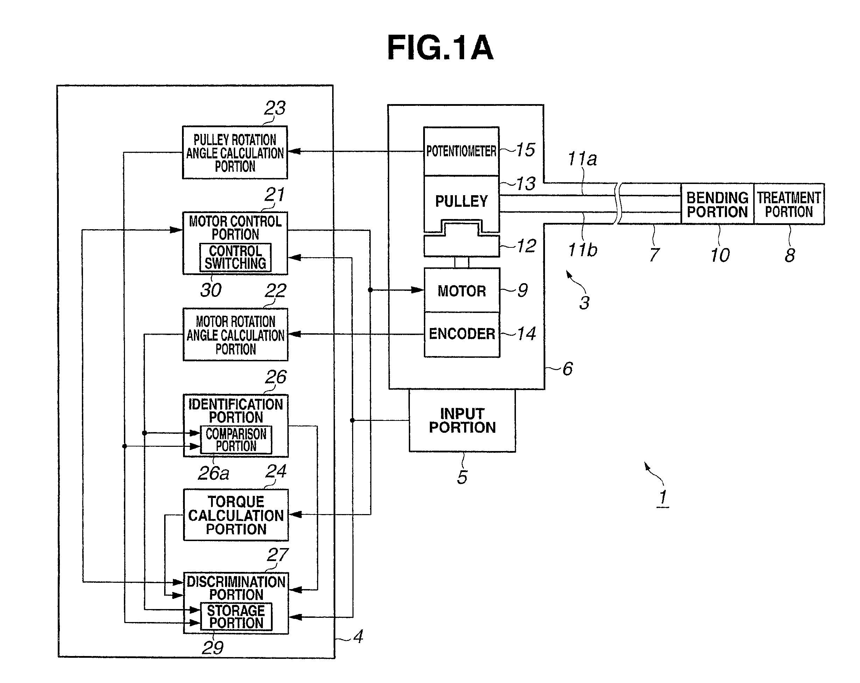

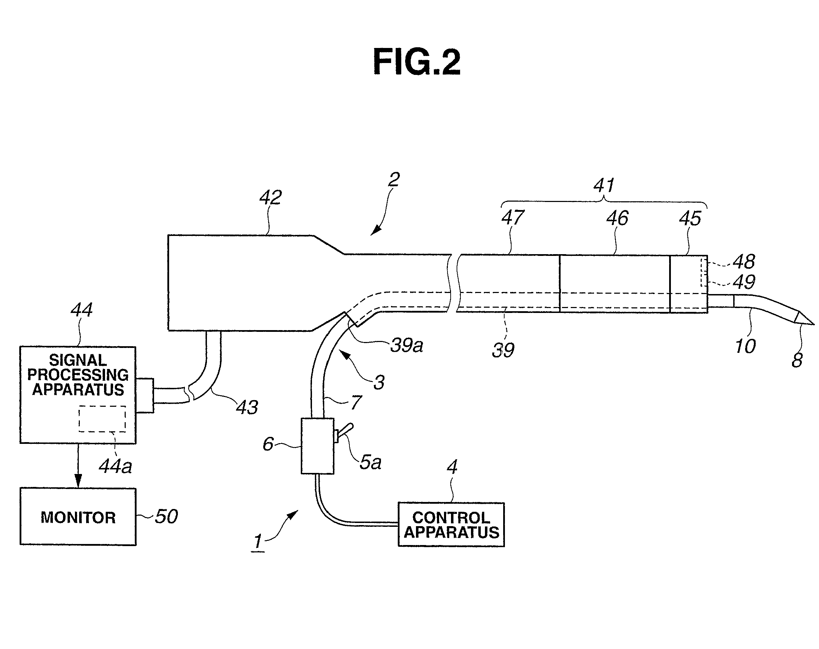

As shown in Fig. 1A, a medical treatment instrument apparatus 1 according to a first embodiment of a medical control apparatus of the present invention includes, for example, an active treatment instrument (hereunder, referred to simply as "treatment instrument") 3 as a medical apparatus that actively drives to bend a bending portion 10, that is inserted through the inside of a treatment instrument channel (hereunder, referred to as "channel") 39 of an endoscope 2 as shown in Fig. 2 that is inserted into a body cavity and used.

The medical treatment instrument apparatus 1 also includes a control apparatus 4 that is connected to the treatment instrument 3 and performs control with respect to the treatment instrument 3, and an input portion 5 that is connected to the control apparatus 4 and with which an operator such as a surgeon performs an operation to input instructions. In the example shown in Fig. 2, the input portion 5 is constituted by a joystick apparatus 5a that is provided on a grasping portion 6 at a rear end of the treatment instrument 3. Note that, the joystick apparatus 5a may also be provided at a location other than the grasping portion 6.

The treatment instrument 3 includes a shaft portion 7 as a flexible and elongated insertion portion that is inserted through the inside of the channel 39, a treatment portion 8 that performs treatment and is provided at a distal end of the shaft portion 7, and a motor 9 as a drive portion that is provided at a rear end of the shaft portion 7. Note that, in the specific example shown in Fig. 2, the treatment portion 8 is constituted by, for example, a biopsy needle that extracts a sample of living tissue by puncturing a diseased part or the like. Further, in Fig. 2, the motor 9 that constitutes a drive portion is provided inside the grasping portion 6.

The bending portion 10 that is capable of bending is provided as an active mechanism at a rear end position of the treatment portion 8. The bending portion 10 is connected through a connecting portion 12 and a pulley 13 constituting a pulling portion with a motor 9 that generates a rotational driving force as a driving force through a pair of angle wires (abbreviated as simply "wires") 11a and 11b for causing the bending portion 10 to bend.

Note that, the wires 11a and 11b are inserted through the inside of the shaft portion 7 in a slackened state. The bending portion 10 is formed using an elastic member that has elastic force that is described later.

By pulling and driving one of the pair of wires 11a and 11b and slackening the other of the pair of wires 11a and 11b in accordance with the torque as a generated rotational driving force, the motor 9 drives the bending of the bending portion 10 toward the side of the wires 11a and 11b. Note that, since the shaft portion 7 is formed in an elongated shape, the bending portion 10 is formed on a distal end side of the shaft portion 7.

The motor 9 includes an encoder 14 as detection means that detects a rotational driving amount (or motor rotation angle) as a driving amount of the motor 9. The encoder 14 is constituted by a rotary encoder or the like.

Further, a potentiometer 15 is attached to the pulley 13 constituting a pulling portion that is rotatably connected through the connecting portion 12 with the motor 9. The potentiometer 15 serves as detection means that detects a pulley rotational driving amount or a pulley rotation angle as a pulling portion driving amount (or pulling and driving amount) of the pulley 13. Note that, a configuration may also be adopted that detects a pulley rotational driving amount or a pulley rotation angle using a rotary encoder instead of the potentiometer 15.

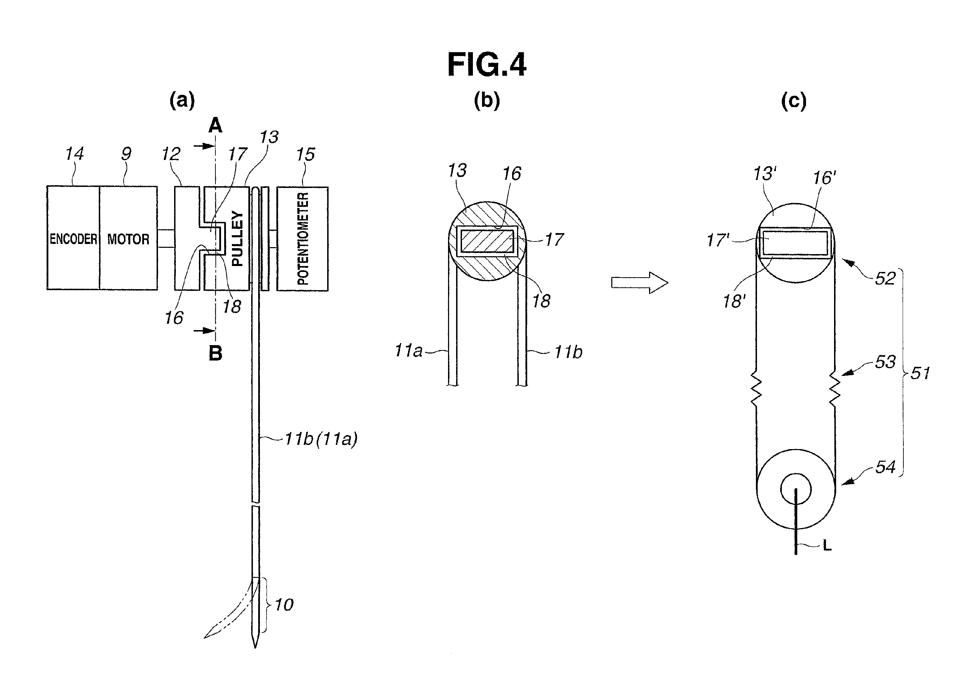

The connecting portion 12 that connects the motor 9 and the pulley 13 without an intervening wire includes, as shown in Fig. 4(A) and Fig. 4(B), a rectangular concave portion 16 that is provided (integrally with the pulley 13) in the center of the disk-shaped pulley 13, and a convex portion 17 that is connected to a rotation shaft of the motor 9 and is engagingly inserted into the concave portion 16.

The connecting portion 12 also includes a backlash portion (or play portion) 18 that generates a positional relationship which cannot transmit (does not transmit) a rotational driving force from the motor 9 between the concave portion 16 and the convex portion 17. Note that, Fig. 4(B) shows a cross-sectional view along a line A-B in Fig. 4(A).

The connecting portion 12 has a positional relationship that transmits a rotational driving force (torque) from the motor 9 as a result of contact between an engagement surface of an inner face of the concave portion 16 and an engagement surface of a side face of the convex portion 17, and also enters a state of a positional relationship that cannot transmit the rotational driving force in a state in which the two engagement surfaces do not contact against each other in a rotational direction of the motor 9 by means of the backlash portion 18.

In the present embodiment, the connecting portion 12 is configured to directly connect the motor 9 and the pulley 13 within a short distance (more specifically, approximately coaxially) without an intervening wire (which is long and with which a time delay arises when transmitting a driving force), and thus a decline in responsiveness is prevented.

Note that, the present invention is not limited to a configuration in which the concave portion 16 is integrally provided in the pulley 13. A configuration may also be adopted in which the concave portion 16 is provided on the motor 9 side, and the convex portion 17 is provided on the pulley 13 side.

As shown in Fig. 1A, the control apparatus 4 includes a motor control portion 21 that performs control that drives the motor 9, and a motor rotation angle calculation portion 22 that calculates or detects a motor rotation angle (motor rotation amount) based on a detection signal with respect to a rotational driving amount or a motor rotation angle of the motor 9 that is outputted from the encoder 14.

The control apparatus 4 also includes a pulley rotation angle calculation portion 23 that calculates or detects a pulley rotation angle based on a detection signal with respect to a pulley rotation angle from the potentiometer 15.

In addition, the control apparatus 4 includes a comparison portion 26a that compares a change amount of the motor rotation angle and a change amount of the pulley rotation angle, and an identification portion 26 that, based on a comparison result obtained by the comparison portion 26a, identifies a boundary between a range in which it is possible for a restoring force of the elastic member forming the bending portion 10 to contribute to bending of the bending portion 10 (contribution possible range) and a range in which it is not possible for the restoring force to contribute to bending of the bending portion 10 (contribution impossible range) due to the occurrence of slackness in the wires 11a and 11b.

Note that, although a configuration example is shown in Fig. 1A in which an output signal of the motor rotation angle calculation portion 22 and an output signal of the pulley rotation angle calculation portion 23 are inputted to the comparison portion 26a, a configuration may also be adopted in which, instead, output signals of the encoder 14 and the potentiometer 15 are inputted.

Further, although in Fig. 1 A a configuration is shown in which the identification portion 26 incorporates the comparison portion 26a, a configuration may also be adopted in which the comparison portion 26a is provided outside the identification portion 26.

In plainer terms, the aforementioned boundary is a boundary position or a boundary state that, when using the wires 11a and 11b that have some slackness, serves as a boundary between a case where bending of the bending portion 10 stops because of a restoring force and a case where slackness in the wires 11a and 11b substantially occurs.

Further, in the present embodiment, a configuration is adopted that can identify the aforementioned boundary even in a situation in which a restoring force acts, and utilizes the identified information to facilitate control to deal with the occurrence of slackness and also to make it easier to correspond to driving for bending that exhibits a hysteresis characteristic.

Note that, although a boundary between the above described contribution possible range and contribution impossible range can be identified by the identification portion 26, in order to correspond to a case where a control method of the motor 9 is changed (switched) in a state in which slackness has occurred, the control apparatus 4 includes a torque calculation portion 24 that calculates a torque as a rotational driving force of the motor 9 based on a characteristic of a drive current or a drive voltage that drives the motor 9.

The control apparatus 4 also includes a discrimination portion 27 that discriminates a bending state of the bending portion 10 based on a torque calculated by the torque calculation portion 24 or the like, as a result of a boundary being identified by the identification portion 26. The discrimination portion 27 outputs the discrimination result to the motor control portion 21.

The motor control portion 21 performs control to rotationally drive the motor 9 in accordance with the bending state discriminated by the discrimination portion 27. Note that, a configuration may also be adopted in which information of a result of identification of a boundary by the identification portion 26 is outputted to the motor control portion 21 that forms a control portion without passing through the discrimination portion 27, and the motor control portion 21 changes (switches) the control method for driving the motor 9 (that pulls the wires 11a and 11b) based on the information.

Further, although a configuration in which the motor control portion 21 and the discrimination portion 27 are separate blocks is shown in Fig. 1A, a configuration may also be adopted in which the motor control portion 21 includes the discrimination portion 27.

Based on the identified result from the identification portion 26, the motor control portion 21 can recognize a specific bending state position as a specific bending state that the bending portion 10 has reached at the aforementioned boundary under the restoring force, and can change the control method of the motor 9 or the like. In other words, identification of the boundary can also be said to be identification of a bending state at a specific bending state position when the bending portion 10 has bent as far as the specific bending state position as a specific bending state that is the aforementioned boundary to which bending portion 10 is bent under the restoring force, without applying a torque as a driving force that bends the bending portion 10 to the bending portion 10 from the motor 9 as a drive portion. Note that, in the specific bending state position, the bending portion holds (maintains) the bending state.

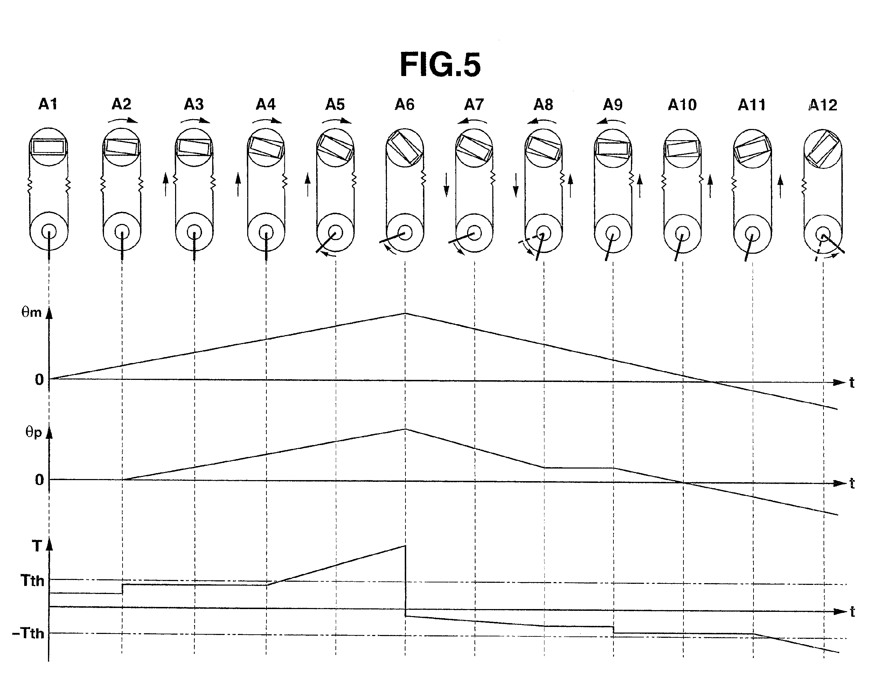

Note that, when shown in Fig. 5 that is described later, the aforementioned boundary corresponds to a state from reference symbol A8 to A9 or a state at an arbitrary position from reference symbol A8 to A9 (in particular, the main position is the specific position denoted by reference symbol A8 that is the initial point of the boundary). In this case, because of a characteristic that a change amount of the pulley rotation angle is 0, identification of this boundary is easy.

On the other hand, although a state corresponding to a state from reference symbol A8 to A9 that is the same as the state of the boundary can also be regarded as the aforementioned specific bending state position, in the present embodiment a bending state position in the state denoted by reference symbol A8 is assumed to be the aforementioned specific bending state position.

In addition, the control apparatus 4 includes, for example, within the discrimination portion 27, a storage portion 29 that memorizes (stores) respective pieces of information regarding a bending angle of the bending portion 10, a motor rotation angle of the motor 9, a pulley rotation angle of the pulley 13 and the like as mutually-associated characteristics information.

Note that, the storage portion 29 may also be configured to also associate and store a torque of the motor 9 as characteristics information. The discrimination portion 27 and the motor control portion 21 refer to the characteristics information of the storage portion 29 as necessary. The storage portion 29 may be provided outside the discrimination portion 27.

The storage portion 29 is connected to the motor control portion 21, the motor rotation angle calculation portion 22, the pulley rotation angle calculation portion 23, the identification portion 26, the torque calculation portion 24, the input portion 5 and the like.

Further, in addition to storing the above described characteristics information, the storage portion 29 chronologically (on a time-series basis) stores information such as a bending angle of the bending portion 10, a motor rotation angle of the motor 9, a pulley rotation angle of the pulley 13, a torque, an identification result of the identification portion 26, a discrimination result obtained by the discrimination portion 27, information relating to control switching by the motor control portion 21, a bending instruction value from the input portion 5 and the like.

Note that, information regarding a bending angle of the bending portion 10 is previously associated with information regarding a pulley rotation angle or a motor rotation angle and stored in the storage portion 29, and the information regarding a bending angle of the bending portion 10 is also updated in a time-series manner based on the information in time series regarding the pulley rotation angle or the motor rotation angle.

In response to input of an instruction to bend the bending portion 10 from the input portion 5, the motor control portion 21 performs control to apply (supply) a motor drive signal to the motor 9 to rotationally drive the motor 9.

When performing control to rotationally drive the motor 9, the motor control portion 21 performs control that rotationally drives the motor 9 by switching the driving speed (motor rotation speed) thereof in accordance with the three bending states based on the discrimination result obtained by the discrimination portion 27. That is, the motor control portion 21 includes a control switching portion 30 that performs control for switching the rotation speed according to the three bending states.

Note that, an instruction that is inputted from the input portion 5 is also inputted to the storage portion 29 provided inside the discrimination portion 27 as described above, and information regarding the inputted instruction is also stored on a time-series basis in the storage portion 29.

Further, when the motor control portion 21 performs control that rotationally drives the motor 9 by means of a motor drive signal based on the discrimination result obtained by the discrimination portion 27, the storage portion 29 performs a correction operation if correction (updating) of characteristics information is required, and updates the characteristics information (under control by the discrimination portion 27). Note that, a configuration may also be adopted in which correction and updating of characteristics information of the storage portion 29 is performed under control of the motor control portion 21 instead of being performed under control of the discrimination portion 27.

Specifically, according to the discrimination result obtained by the discrimination portion 27, in a driving state in which slackness occurs in the wires 11a and 11b, to remove the slackness in a short time, the motor control portion 21 outputs a motor drive signal to the motor 9 to cause the motor 9 to rapidly rotate so as to take up the slackness in the wires 11a and 11b by means of the pulley 13, and also corrects and updates information regarding the motor rotation angle of the motor 9 and the pulley rotation angle of the pulley 13 with respect to the bending angle of the bending portion 10 by an amount corresponding to the amount of the slackness.

By correcting the information in this manner, even in a case in which slackness has arisen in the wires 11a and 11b, characteristics information regarding the bending angle of the bending portion 10 and the motor rotation angle and pulley rotation angle can be maintained so as to match the actual driving state (operating state). Furthermore, in a case where bending has been repeated also, the bending portion 10 can be subjected to accurate bending control so as to enter a state of a bending angle that has been instructed by an input operation from the input portion 5.

Further, in a case where a backlash occurs and the backlash is eliminated, the storage portion 29 updates the characteristics information with respect to the motor rotation angle and the pulley rotation angle that corresponds to that portion.

Thus, in a case where hysteresis has occurred also, a configuration is adopted such that the characteristics information is updated to characteristics information that corresponds to the hysteresis, and accurate and favorable bending control can be performed.

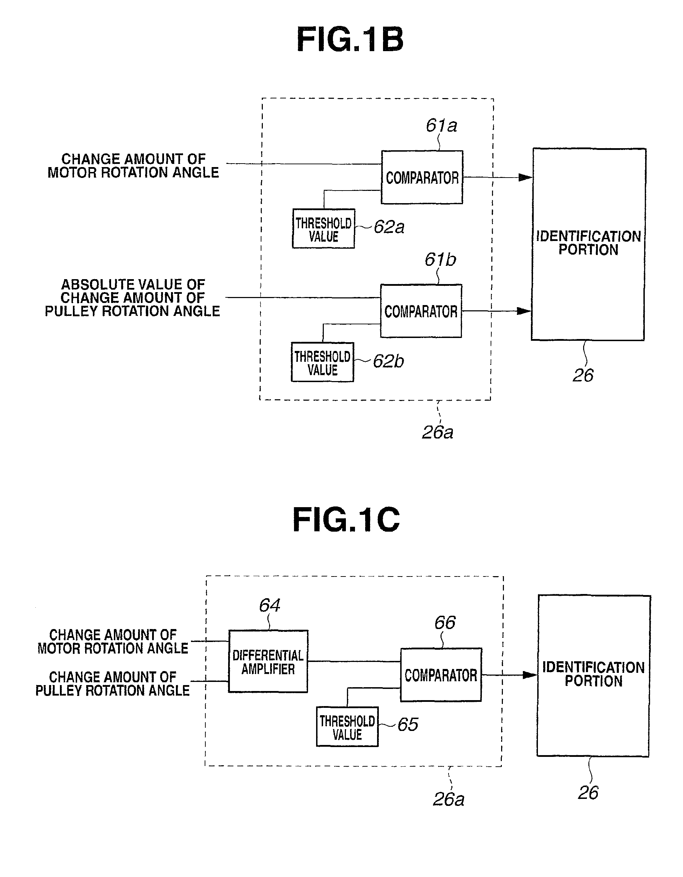

Fig. 1B shows a configuration example of a case in which the comparison portion 26a and the identification portion 26 are separate elements.

The comparison portion 26a includes a first comparator 61a and a window-type second comparator 61b. The comparison portion 26a outputs a comparison result of the first comparator 61a and the second comparator 61b to the identification portion 26.

A change amount of a motor rotation angle and a first threshold value 62a that is used for judging the change amount of the motor rotation angle are inputted to the first comparator 61a. The first comparator 61 a outputs a result of comparison thereof to the identification portion 26.

Further, an absolute value of a change amount of the pulley rotation angle, and a second threshold value 62b that is used for judging the absolute value of the change amount are inputted to the second comparator 61b. The second comparator 61b outputs a result of comparison thereof to the discrimination portion 27. For example, the second comparator 61b outputs a positive comparison result if the absolute value of the change amount of the pulley rotation angle is within the second threshold value 62b, and outputs a negative comparison result if the absolute value of the change amount of the pulley rotation angle is outside the second threshold value 62b. Note that, a circuit that calculates an absolute value of a change amount of the pulley rotation angle based on the change amount is provided at a stage prior to the comparator 61b (not shown).

A change amount of the motor rotation angle is calculated based on a motor rotation angle that changed per short predetermined time period ta. A change amount of the pulley rotation angle is also calculated in a similar manner based on a pulley rotation angle that changed per short predetermined time period ta.

The first threshold value 62a, for example, is set to a value that corresponds to a change characteristic of a motor rotation angle θm as shown in Fig. 5 that is described later. Note that, Fig. 5 shows a motor rotation angle θm and a pulley rotation angle θp and the like that correspond to a bending angle of the bending portion 10 in a case where the bending portion 10 has been bent.

More specifically, the first threshold value 62a is set in correspondence with a change characteristic of the motor rotation angle θm that corresponds to a state from reference symbol A6 to A8 in Fig. 5. In this case, the first threshold value 62a is set in correspondence with a characteristic that the motor rotation angle θm decreases over time, and is set to a value that includes an allowable margin from the characteristic shown in Fig. 5.

In other words, even if the motor rotation angle θm changes so as to decrease with a characteristic that deviates to some extent from the characteristic shown in Fig. 5, the first comparator 61a outputs a comparison result (for example, an output signal with positive polarity) to the effect that the motor rotation angle θm is changing by a change amount that is within a permitted range.

The second threshold value 62b, for example, is set to a value that corresponds to a change in the pulley rotation angle θp shown in reference symbol A8 to A9 in Fig. 5. In this case, to enable judgment of a state in which the pulley rotation angle θp does not change and maintains a constant value as shown by the state between reference symbols A8 and A9, the second threshold value 62b, for example, is set to a positive value that is close to 0 (that is less than an absolute value of a negative change amount in the case of the state between reference symbols A7 and A8).

The second comparator 61b judges whether or not an absolute value of a change amount of the pulley rotation angle is within the second threshold value 62a.

That is, the second comparator 61b is a window-type comparator as described above, and detects whether or not the absolute value of the change amount of the pulley rotation angle is a value close to 0 by performing a comparison with the second threshold value 62b.

The second comparator 61b, for example, outputs a positive output signal when the absolute value of a change amount of the pulley rotation angle is within the second threshold value 62b, and outputs a negative output signal in other cases (when the absolute value is not within the second threshold value 62b).

Therefore, the second comparator 61b outputs an output signal with negative polarity in a case of the state from reference symbol A7 to immediately before A8, and outputs an output signal with positive polarity from the state denoted by reference symbol A8 onwards. Based on the comparison outputs of the first comparator 61a and the second comparator 61b, the identification portion 26 identifies (discriminates) that the bending portion 10 has been bent in a boundary state by the restoring force when the output signal of the first comparator 61a is positive and the output signal of the second comparator 61b has changed from negative to positive.

Based on the comparison outputs of the comparison portion 26a received through the discrimination portion 27, or without passing through the discrimination portion 27, the motor control portion 21 performs control that changes (switches) the control method of the motor 9. In other words, the motor control portion 21 performs control that changes (switches) a pulling method for pulling the wires 11a and 11b through the pulley 13 forming the pulling portion.

Note that, a state in which a state where a change amount of the pulley rotation angle is 0 continues from reference symbol A8 to reference symbol A9 in Fig. 5 corresponds to a bending state in which the motor 9 and the pulley 13 do not engage due to the backlash portion 18, that is, a backlash state.

The aforementioned backlash state also includes a state from reference symbol A1 to A2 in which the pulley rotation angle θp is a value that does not change in Fig. 5, and a configuration may be adopted that enables detection thereof by the comparison portion 26a.

In that case, if the motor rotation angle changes, by utilizing a comparison result of the second comparator 61b such that a change amount of the pulley driving amount is within the threshold value 62b that is close to 0, the comparison portion 26a can also detect the backlash state from reference symbol A1 to A2. However, in this case, it is good that the configuration is adapted in which the first comparator 61 a side also detects a case where a change amount of the motor rotation angle is positive, that is, is constituted by a window-type comparator.

A configuration such as the modification shown in Fig. 1C may be adopted instead of the configuration in Fig. 1B. This modification is also a configuration example for identifying a state from reference symbol A8 to A9 in Fig. 5. The comparison portion 26a includes a differential amplifier 64 that compares a change amount of the motor rotation angle and a change amount of the pulley rotation angle and outputs a difference value, and a comparator 66 that compares the difference value and the threshold value 65. An output signal of the comparator 66 is inputted to the identification portion 26.

The differential amplifier 64 outputs a first difference value as a difference value between a change amount of the motor rotation angle and a change amount of the pulley rotation angle for a state from reference symbol A7 to immediately before reference symbol A8, and the first difference value becomes a second difference value as a difference value that is only a change amount of the motor rotation angle from reference symbol A8 onwards (up to S9).

Therefore, the threshold value 65 for enabling judgment with respect to a case in which there is only a change amount of the motor rotation angle is, for example, set to an intermediate value between the first difference value and the second difference value, and a comparison result is inverted with respect to a case where an output signal of the differential amplifier 64 that is inputted to the comparator 66 is the first difference value and a case where the aforementioned output signal is the second difference value. That is, when the bending portion 10 is bent in a boundary state, in the comparison portion 26a, the comparator 66 outputs an output signal whose polarity has been inverted.

Further, when the polarity of the output signal of the comparator 66 (comparison portion 26a) has been inverted, the identification portion 26 identifies (detects) that the bending portion 10 is bent in a boundary state.

From reference symbol A8 to A9, a state in which a change amount of the pulley driving amount is 0 continues. This state corresponds to a bending state in which the motor 9 and the pulley 13 are not engaged due to the backlash portion 18, that is, a backlash state.

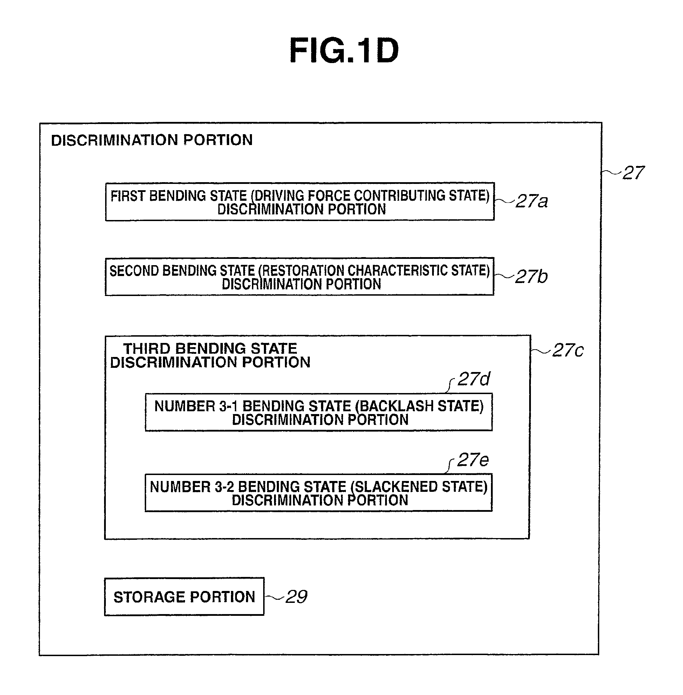

As shown in Fig. 1D, based on the identification result of the identification portion 26 shown in Fig. 1B and the like, by judging whether or not an absolute value of a torque calculated by the torque calculation portion 24 is a predetermined value or more, the discrimination portion 27 discriminates between a driving force contributing state in a case where the absolute value of the torque is a predetermined value or more and a bending state that is not the driving force contributing state in which the absolute value of the torque is less than a predetermined value. Note that, the discrimination portion 27 has a function of a judgment portion that makes a judgment as to whether or not an absolute value of a torque that is calculated by the torque calculation portion 24 is a predetermined value or more.

The discrimination portion 27 includes a first bending state discrimination portion 27a that discriminates a driving force contributing state in a case where the absolute value of a torque is a predetermined value or more as a first bending state.

The discrimination portion 27 also discriminates bending states that are not the driving force contributing state as two bending states, that is, a second bending state and a third bending state, respectively.

In the case of a judgment result to the effect that an absolute value of a torque calculated by the torque calculation portion 24 is less than a predetermined value, the discrimination portion 27 discriminates that the bending state is not the driving force contributing state. Further, the discrimination portion 27 includes a second bending state discrimination portion 27b that discriminates a bending state in which, immediately after shifting to a bending state that is not the driving force contributing state from the driving force contributing state, the bending angle of the bending portion 10 changes as far as the specific bending state position that is the above described boundary when the pulley 13 is rotated by a restoring force of the elastic member without the motor 9 applying a torque to the load side (pulley side) as being the second bending state.

The second bending state corresponds to a state in which the bending portion 10 is bent (until reaching the specific bending state position) by a restoring force produced by the elastic member forming the bending portion 10 in a state in which a torque that bends the bending portion 10 is not generated by the motor 9, when an instruction to perform a bending operation to return the bending portion 10 to the neutral state side is made immediately after the bending portion 10 was bent by a large amount.

Therefore, a function that discriminates a second bending state by means of the discrimination portion 27 corresponds to a function that discriminates a restoration characteristic state (as a characteristic state in which bending is caused by a restoring force).

A state where the bending angle of the bending portion 10 changes in the second bending state is one in which the pulley rotation angle of the pulley 13 changes in a decreasing direction, and hence the discrimination portion 27 discriminates the second bending state based on that change characteristic. Further, the second bending state is maintained until the specific bending state position (denoted by reference symbol A8 in Fig. 5) at which the restoring force becomes 0 (or a state where the restoring force balances with frictional forces or the like).

The discrimination portion 27 also includes a third bending state discrimination portion 27c that discriminates, as a third bending state, a bending state in which a torque value T generated by the motor 9 is not detected as a value that is equal to or greater than a (torque threshold value Tth as a) predetermined value, in a bending state that is not the driving force contributing state excluding the aforementioned second bending state.

The third bending state corresponds to a state where the motor 9 and the pulley 13 re-engage (one engagement is disconnected and another engagement is made) by means of the backlash portion 18 that corresponds to a bending state at the aforementioned specific bending state position, or a state where slackness has arisen in the wires 11a and 11b.

In addition, the discrimination portion 27 includes a number 3-1 bending state discrimination portion 27d and a number 3-2 bending state discrimination portion 27e that further discriminate the third bending state into two bending states, namely, a number 3-1 bending state and a number 3-2 bending state.

That is, the discrimination portion 27 discriminates the number 3-1 bending state in which the pulley rotation angle does not change with respect to a change amount of the motor rotation angle, and the number 3-2 bending state in which the pulley rotation angle also changes with respect to a change amount of the motor rotation angle. Note that, the number 3-1 bending state and the number 3-2 bending state may also be defined as a third bending state and a fourth bending state, respectively.

The aforementioned number 3-1 bending state corresponds to a state in which the motor 9 and the pulley 13 re-engage by means of the backlash portion 18 of the connecting portion 12, in other words, a state (backlash state) in which the motor 9 and the pulley 13 are not engaged in the bending direction. The number 3-2 bending state corresponds to a state in which slackness of the wires 11a and 11b has arisen. That is, the discrimination portion 27 has a function of a backlash state judgment portion that judges a backlash state as the number 3-1 bending state discrimination portion 27d, and a function of a slackened state judgment portion that judges a slackened state of the wires as the number 3-2 bending state discrimination portion 27e.

Thus, the discrimination portion 27 includes the first bending state discrimination portion 27a, the second bending state discrimination portion 27b and the third bending state discrimination portion 27c.

Note that, a boundary where the second bending state ends and the number 3-1 bending state in the third bending shape is entered is the aforementioned specific bending state position, and both two bending states can be discriminated based on information regarding the specific bending state position.

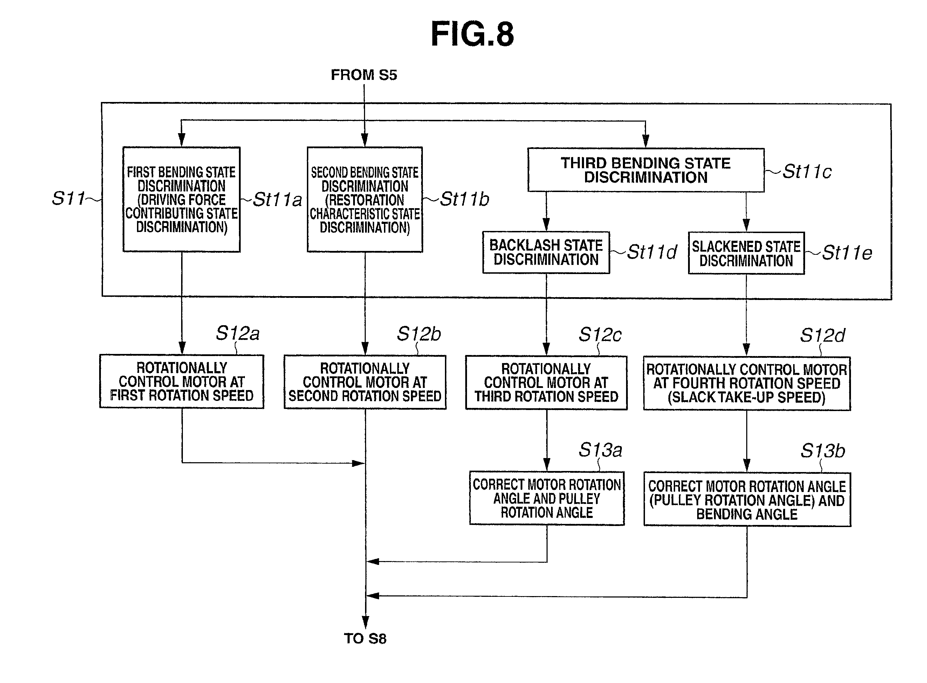

The discrimination portion 27 sends the discrimination result to the motor control portion 21. The motor control portion 21 controls so as to perform rotational driving that switches the rotation speed of the motor 9 in accordance with the discrimination result (specifically, controls as shown in Fig. 8).

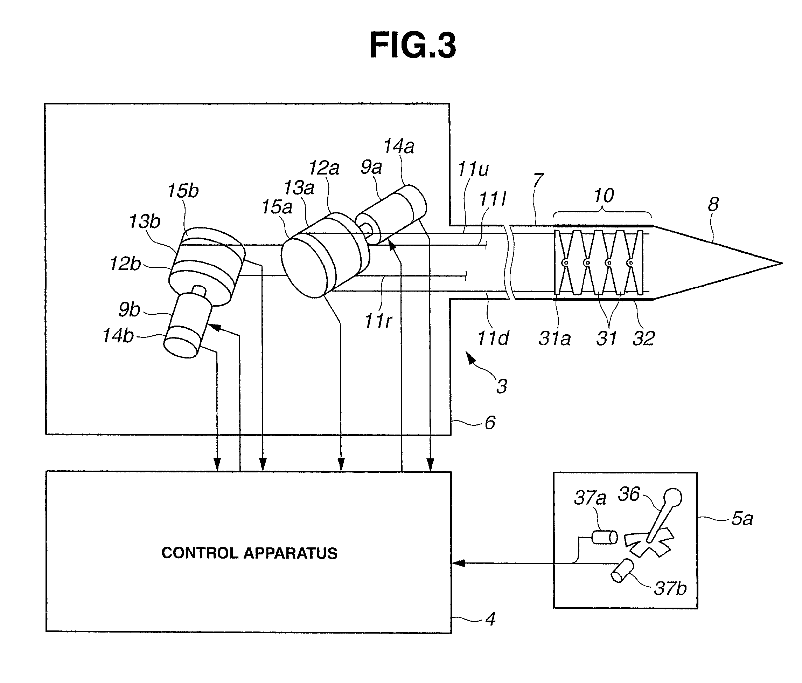

Fig. 3 illustrates a specific configuration example of the treatment instrument 3. As shown in Fig. 3, a biopsy needle that utilizes puncturing is formed as the treatment portion 8 at the distal end of the shaft portion 7. The bending portion 10 is formed at the rear end of the biopsy needle. In the bending portion 10, a plurality of bending pieces 31 are provided that have a substantially annular shape. Portions of the bending pieces 31 that are adjacent to each other in the longitudinal direction of the shaft portion 7 are pivotably connected by rivet portions 31a.

The bending direction of each bending piece 31 is determined by the position at which the rivet 31a is provided. The rivets 31a are disposed at horizontal and vertical positions in an alternating manner or at appropriate cycles, enabling the bending pieces 31 to bend in the vertical and horizontal directions.

Note that, the outer circumferential sides of the bending pieces 31 are covered by an outer sheath member formed by a bending rubber tube 32 as an elastic member (that has an elastic force) that seals and protects the bending portion 10 in a freely bendable manner. By means of the elastic force of the bending rubber tube 32 forming the bending portion 10 or the like, in a case where the bending portion 10 is bent to a particularly large degree, a restoring force arises as an elastic force that attempts to return the bending portion 10 to a neutral state in which the bending portion 10 does not bend, and the bending portion 10 bends to the neutral state side under the restoring force.

When the wires 11a and 11b are short, although the bending portion 10 bends under the restoring force as far as a state that is close to the neutral state, even in a case where the restoring force does not become 0, if the restoring force balances with frictional forces that act between the wires 11a and 11b inserted through the inside of the shaft portion 7 and members surrounding the wires 11a and 11b, bending caused by the restoring force stops at the bending angle of the balanced state.

Note that, Fig. 3 is a simplified view showing only the rivets 31a that cause bending in the vertical direction. Furthermore, wires 11u, 11d and 11l, 11r for bending in the vertical direction and the horizontal direction are inserted through the shaft portion 7, and the distal ends of the wires 11u, 11d and 11l, 11r are fixed to the treatment portion 8.

Furthermore, the rear ends of the wires 11u, 11d and 11l, 11r are looped over a vertical bending pulley 13a and a horizontal bending pulley 13b that are disposed inside the grasping portion 6 whose diameter is extended at the rear end of the shaft portion 7.

The pulleys 13a and 13b are connected to the rotation shafts of motors 9a and 9b, respectively, through connecting portions 12a and 12b in which the above described backlash portion is provided. The motors 9a and 9b are freely rotated forward or backward according to a motor drive signal from the motor control portion 21.

Concurrently with the rotations of the motors 9a and 9b, the respective pulleys 13a and 13b that are connected through the connecting portions 12a and 12b in which the backlash portion 18 is provided also rotate, and the wires 11u, 11d and 111, 11r that are respectively looped over the pulleys 13a and 13b are pulled and slackened, respectively. Thus, the bending portion 10 is driven to bend in the direction of the pulled wire. In addition, encoders 14a and 14b are connected to the rotation shafts of the motors 9a and 9b, respectively, and potentiometers 15a and 15b are connected to the pulleys 13a and 13b, respectively.

Further, the joystick apparatus 5a that, for example, constitutes the input portion 5 includes a joystick 36 that can be tilted arbitrarily in the vertical and horizontal directions, respectively, and encoders 37a and 37b that detect tilting angles of the joystick 36 in the vertical and horizontal directions, respectively. The direction that the joystick 36 is tilted in is the bending instruction direction with respect to the bending portion 10, and the tilting angle is the instruction value for the bending angle of the bending portion 10.

Detection signals from the encoders 37a and 37b are inputted to, for example, the motor control portion 21 in the control apparatus 4. That is, the bending instruction direction and the instruction value for the bending angle are inputted to the motor control portion 21 from the joystick apparatus 5a as bending instruction input means.

The motor control portion 21 refers to characteristics information stored in the storage portion 29 and the like to determine motor rotation angles of the motors 9a and 9b with respect to the instruction value, and rotationally drives the motors 9a and 9b so that the rotation angles of the motors 9a and 9b detected by the encoders 14a and 14b follow the instruction value.

In practice, since slackness arises in the wires 11a and 11b, in the present embodiment, bending states in which there is or is not slackness or the like are discriminated by the discrimination portion 27. Further, since the slackness state is also affected by bending of the bending portion 10 caused by the restoring force, according to the present embodiment, a boundary between a position of a bending state in which bending caused by the restoring force stops and a position of a bending state in which slackness substantially occurs in a state in which bending caused by the restoring force has stopped is identified by the identification portion 26. The motor control portion 21 changes the control method of the motor 9 utilizing the information regarding the boundary.

As shown in Fig. 2, the endoscope 2 includes an insertion portion 41 that is inserted into a body cavity, an operation portion 42 provided at a rear end of the insertion portion 41, and a universal cable 43 that is extended from the operation portion 42. An end portion of the universal cable 43 is detachably connected to a signal processing apparatus 44.

The insertion portion 41 of the endoscope 2 includes a distal end portion 45 provided at a distal end of the insertion portion 41, a freely bendable bending portion 46 provided at a rear end of the distal end portion 45, and a flexible portion 47 that has flexibility and extends from the rear end of the bending portion 46 to the front end of the operation portion 42.

An illuminating window 48 that emits illuminating light and an observation window 49 that is formed adjacent to the illuminating window 48 are provided in the distal end portion 45 of the insertion portion 41.

Further, the channel 39 through which a treatment instrument can be inserted is provided in the insertion portion 41. A rear end of the channel 39 opens as a treatment instrument insertion port 39a in the vicinity of the front end of the operation portion 42. The operator such as a surgeon can insert the treatment instrument 3 from the treatment instrument insertion port 39a to perform treatment under observation with the endoscope 2.

Further, the signal processing apparatus 44 incorporates a signal processing circuit 44a that generates a video signal based on a signal that is picked up by an unshown objective lens disposed in the observation window 49 and an image pickup device disposed at an image formation position thereof. A video signal generated by the signal processing circuit 44a is outputted to a monitor 50 as a display apparatus. A picked-up image that has been picked up by the image pickup device is displayed as an endoscopic image on a display surface of the monitor 50.

In the present embodiment, the bending portion 10, the pulley 13 that is rotatably suspended via the bending portion 10 and the wires 11a and 11b, and the motor 9 connected through the connecting portion 12 with the pulley 13 that are shown in Fig. 4(A) and Fig. 4(B), are illustrated more simply by a schematized model 51 shown in Fig. 4(C).

Note that, the wires 11a and 11b shown in Fig. 4 represent the wires 11u and 11d or the wires 11l and 11r in Fig. 3. Furthermore, the motor 9 in Fig. 4 corresponds to the motor 9a or 9b in Fig. 3, the pulley 13 in Fig. 4 corresponds to the pulley 13a or 13b in Fig. 3, and the connecting portion 12 in Fig. 4 corresponds to the connecting portion 12a or 12b in Fig. 3.

In the model 51 shown in Fig. 4(C), the connecting portion 12 between the motor 9 and the pulley 13 of Fig. 4(B) is represented by a connecting portion model 52 that has a backlash, the wires 11a and 11b that have slackness are represented by a wire model 53 in which slackness is schematized by a spring, and the bending portion 10 to which the distal ends of the wires 11a and 11b are attached is represented by a bending portion model 54 with concentric circles.

In the connecting portion model 52, the pulley 13 in Fig. 4(B) is represented by a circular pulley model 13', the concave portion 16 of the pulley 13 is represented by a concave portion model 16', the convex portion 17 of the connecting portion 12 is represented by a rectangular convex portion model 17', and the backlash portion 18 is represented by a backlash portion model 18'.

Further, slackness in the wires 11a and 11b in Fig. 4(A) and Fig. 4(B) is represented by schematized wire models 11a' and 11b' (the wire models 11a' and 11b' are represented by the wire model 53) that are represented by spring patterns. In the wire model 53, a wire state of a portion without slackness is shown by a linear wire model, and a wire state of a portion with slackness is shown by wire model that has a spring pattern.

Further, in the bending portion model 54 with concentric circles which models a bending state of the bending portion 10, a bending state of the bending portion 10 is represented in virtual form by a bending direction line L. For example, in a state in which the bending direction line L extends downward in a straight line from the concentric circles, the bending portion 10 is in a neutral state in which the bending portion 10 does not bend in the vertical direction (or horizontal direction).

The medical treatment instrument apparatus 1 of the present embodiment as a medical control apparatus according to the above described configuration includes: the shaft portion 7 as an insertion portion that is provided in the treatment instrument 3 as a medical apparatus and in which the bending portion 10 that is formed using the bending rubber tube 32 as an elastic member having flexibility is provided on a distal end side; the motor 9 as a drive portion that generates a driving force for subjecting the bending portion 10 to a bending operating; the wires 11a and 11b that are inserted through the inside of the insertion portion and provided with slackness and connected to the bending portion 10; the pulley 13 as a pulling portion that pulls the wires 11a and 11b; the connecting portion 12 that connects the drive portion and the pulling portion so as to have a positional relationship that transmits the driving force between the drive portion and the pulling portion and a positional relationship that cannot transmit the driving force between the drive portion and the pulling portion; the encoder 14 as a driving amount detection portion that detects a driving amount of the drive portion as a drive portion driving amount; the potentiometer 15 as a pulling portion driving amount detection portion that detects a pulling portion driving amount that is pulled by the pulling portion; a comparison portion 26a that compares a change amount of the drive portion driving amount and a change amount of the pulling portion driving amount; and an identification portion 26 that, based on a comparison result obtained by the comparison portion 26a, identifies a boundary between a range in which it is possible for a restoring force of the elastic member forming the bending portion 10 to contribute to bending of the bending portion 10 and a range in which it is not possible for the restoring force to contribute to bending of the bending portion 10 because of the occurrence of slackness in the wires 11a and 11b.

Next, operations of the medical treatment instrument apparatus 1 of the present embodiment are described.

In Fig. 5, reference symbols A1 to A12 denote representative bending states when performing operations in which the motor 9 shown in Fig. 4 is rotationally driven to rotate the bending portion 10 by a predetermined angle via the pulley 13 that is connected to the motor 9 through the connecting portion 12, and thereafter the bending portion 10 is rotated by an appropriate angle in the opposite direction.

Note that, in Fig. 5, representative bending states when the bending portion 10 is driven to bend using the model shown in Fig. 4 are denoted by reference symbols A1 to A12 in the uppermost section, the corresponding motor rotation angles θm are shown in the second section, pulley rotation angles θp are shown in the third section, and torque values T of the motor 9 that are calculated by the torque calculation portion 24 are shown in the fourth section.

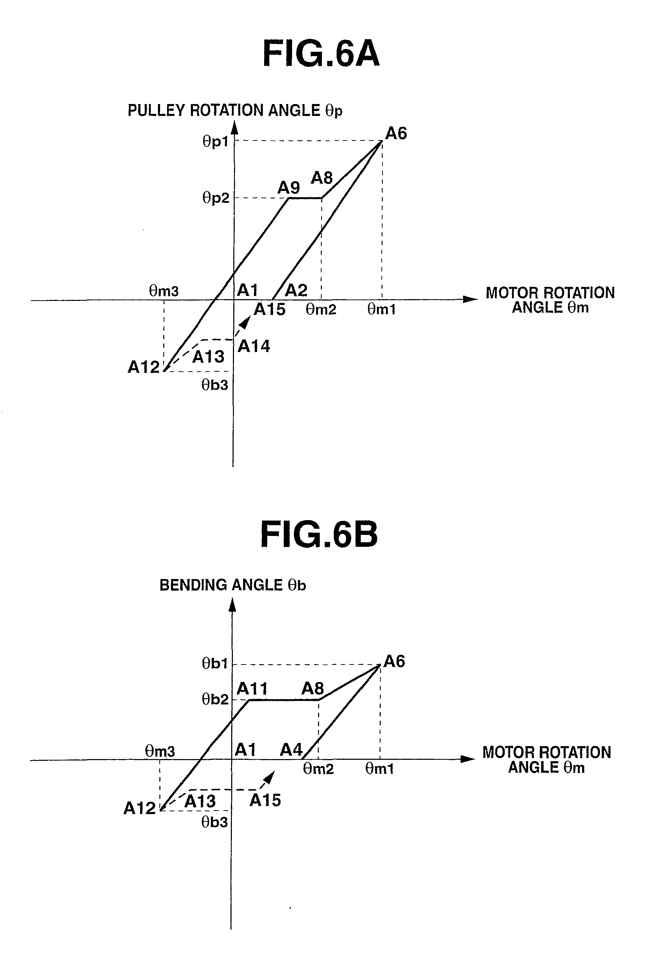

Further, in correspondence with Fig. 5, Fig. 6A and Fig. 6B illustrate that the relationship between characteristics of the motor rotation angle θm and the pulley rotation angle θp, and between the bending angle θb of the bending portion 10 and the motor rotation angle θm each have a characteristic with hysteresis.

The state denoted by reference symbol A1 is a state in which the two engagement surfaces of the motor 9 and the pulley 13 are not engaged by means of the connecting portion 12 (backlashed engagement), and represents a case of a neutral state in which there is slackness in the wires 11a and 11b.

In the state denoted by reference symbol A1, when the motor 9 is rotated in the direction indicated by an arrow that is shown in Fig. 5, the motor rotation angle θm increases from 0, and in the state denoted by reference symbol A2, when a state is entered in which the motor 9 and the pulley 13 have engaged by means of the connecting portion 12, together with rotation of the motor 9 as denoted by reference symbols A2 and A3, the pulley 13 also rotates and the pulley rotation angle θp increases from 0.

In other words, in the state denoted by reference symbols A1 to A2, the pulley rotation angle θp does not change even though the motor 9 rotates and the motor rotation angle θm thereof changes. The relationship characteristic in this case is as shown in Fig. 6A.

In the state denoted by reference symbol A2, since there is slackness in the wire 11a in the pulling direction, in the state denoted by reference symbol A3 in which the motor 9 and the pulley 13 have been rotated further, the bending angle θb of the bending portion 10 does not change from the angle 0 that is the neutral state.

Further, this state continues until the state denoted by reference symbol A4, that is, a state in which the slackness of the wire 11a is removed or cleared. When the motor 9 and pulley 13 rotate to pass the state denoted by reference symbol A4 and reach the state denoted by reference symbol A5, the bending portion 10 bends to the side of the pulled wire 11a.

As shown in the lowermost section in Fig. 5, |T|<Tth in the vicinity of the states denoted by reference symbols A1 to A4, and based on a torque calculated by the torque calculation portion 24, the discrimination portion 27 judges that the bending state is not a driving force contributing state in which the bending portion 10 is driven to bend by the motor 9.

In contrast, |T|≥Tth in the state denoted by reference symbol A5 that is beyond the state denoted by reference symbol A4. Therefore, the discrimination portion 27 judges that the state is the driving force contributing state, and also discriminates that the bending state is the first bending state as the driving force contributing state that contributes to driving to bend the bending portion 10.

When the motor 9 is rotated to exceed the state denoted by reference symbol A5 and reach a state of a predetermined motor rotation angle θml denoted by reference symbol A6, the pulley 13 rotates in conjunction with the rotation of the motor 9, and the bending portion 10 also bends so that a state with a pulley rotation angle θp1 and a bending angle θb1 is entered.

The relationship between the motor rotation angle θm and the pulley rotation angle θp in this case is as shown in Fig. 6A, and the relationship between the motor rotation angle θm and the bending angle θb is as shown in Fig. 6B.

After reaching the predetermined motor rotation angle θml in this manner, if the motor 9 is rotated in the opposite direction to attempt to bend the bending portion 10 in the opposite direction, even in a state in which a driving signal is not actually applied (supplied) so as to rotate the motor 9, as shown in the state denoted by reference symbol A7, the bending portion 10 bends in the direction of the neutral state position by means of a restoring force of an elastic member such as the bending rubber tube 32 as an outer sheath member constituting the bending portion 10.

The restoring force differs in accordance with the size of the bending angle θb 1 and a material of the outer sheath member and the like. Particularly, after the bending portion 10 has been bent by a large amount, the restoring force acts with a large force when bending the bending portion 10 in the opposite direction. Note that, as shown in the states denoted by reference symbol A6 to reference symbol A7 and reference symbol A8, when the bending portion 10 bends in the opposite direction under the restoring force, some of the slackness of the wire 11b is reduced by rotation of the pulley 13.

When rotating the motor 9 in the opposite direction in this manner (direction that decreases the bending angle θb), in a state in which the restoring force is acting, a load with respect to the motor 9 becomes less than in a state without a load (A1-A2) or in a state where slackness of the wire is taken up so as to remove the slackness (A2-A4) (these states are referred to collectively as "state equivalent to no-load").

When the restoring force acts, as shown in the state denoted by reference symbol A8, as a result of the restoring force and frictional forces that act on the wire 11a in resistance to the restoring force and the like, the bending portion 10 bends as far as a specific bending state position that as a specific bending state in which the restoring force that bends the bending portion 10 is substantially 0, and the bending portion 10 maintains the state of that bending angle θb2.

In the bending angle θb2 state, since the motor 9 and the pulley 13 are not in an engagement state in which the motor 9 causes the pulley 13 to rotate in the opposite direction, until reaching the state denoted by reference symbol A9 as an engagement state in which the motor 9 and the pulley 13 re-engage from the state denoted by reference symbol A8, even if the motor 9 rotates in the opposite direction, the pulley 13 does not rotate and maintains a fixed pulley rotation angle θp2 value that does not change.

Therefore, for example, by means of the configuration shown in Fig. 1B or the like, the comparison portion 26a monitors a change amount of the motor rotation angle θm and a change amount of the pulley rotation angle θp, and in a state in which the motor rotation angle θm is changing, can simply detect the specific bending state position by detecting a state in which the change amount of the pulley rotation angle θp is a change from a negative value to 0, for example, a case where the threshold value 62b that is close to 0 and (the absolute value of) the change amount of the pulley rotation angle θp are compared and the change amount of the pulley rotation angle θp is within the threshold value 62b.

The specific bending state position is a boundary between the second bending state and the third bending state. In the present embodiment, a result of this detection is utilized for control of driving to bend.

When the motor 9 is rotated further to pass the state denoted by reference symbol A9, the pulley 13 also rotates together with rotation of the motor 9. However, in this state, since there is slackness in the wire 11b, until the slackness is removed, that is, until reaching the state denoted by reference symbol A11, the bending portion 10 does not change from the bending angle θb2 in the state denoted by reference symbol A8.

Further, if the motor 9 is rotated to pass through the state denoted by reference symbol A11, together with rotation of the pulley 13 that accompanies rotation of the motor 9, the bending angle 8b of the bending portion 10 also changes. The bending portion 10 is bent as far as an appropriate bending angle θb3 in the opposite direction, as denoted by reference symbol A12. Note that, in Fig. 6A and Fig. 6B, the motor rotation angle corresponding to the bending angle θb3 in the state denoted by reference symbol A12 is denoted by θm3, and the pulley rotation angle corresponding thereto is denoted by θp3.

As shown in Fig. 6B, when the bending portion 10 bends in the opposite direction from the bending angle 8b3, since in general the absolute values of the bending angles θb1 and θb3 are different, the size of a restoring force differs according to the size of the absolute value of the bending angle θb3. Consequently, as shown by the dotted lines in Fig. 6A and Fig. 6B, hysteresis characteristics (that do not close) as denoted by reference symbols A13, A14 and A15 are exhibited in accordance with the restoring force in the relevant case.

However, in a case with such a hysteresis characteristic also, by identifying a specific bending state that is generated at a specific bending state position or the like, the respective states of the bending angle 8b of the bending portion 10, the motor rotation angle θm and the pulley rotation angle 8p can be associated and ascertained.

For example, in Fig. 6A, a state denoted by reference symbols A8 to A9 is a state with a characteristic that the change amount of the pulley rotation angle θp2 does not change even if the motor rotation angle θm changes, and a state denoted by reference symbols A13 to A14 exhibits a similar characteristic.

Consequently, by temporally monitoring this state and storing the relevant information in the storage portion 29, characteristics information that reflects a characteristic thereof can be maintained.

By calculating (detecting) temporal changes in operations of the motor 9 and the pulley 13 and the like in this manner and storing information regarding the changes in the storage portion 29, even if the operating state of the motor rotation angle θm and the pulley rotation angle θp exhibits a characteristic with hysteresis, the positional relationship denoted by reference symbol A14 or the like can be ascertained based on the relationship between the two angles, for example, the relationship between the motor rotation angle θm and the pulley rotation angle θp in the state denoted by reference symbol A13.

Thus, according to the present embodiment, the discrimination portion 27 discriminates each of a third bending state (number 3-1 bending state) denoted by reference symbols A1-A2 and A8-A9 in Fig. 5, a third bending state (number 3-2 bending state) denoted by reference symbols A2-A4 and A9-A11 in Fig. 5, a first bending state denoted by reference symbols A4-A6 and A11-A12 in Fig. 5, and a second bending state denoted by reference symbols A6-A8 in Fig. 5.

Accordingly, even when behavior of the bending angle of the bending portion 10, the motor rotation angle θm and the pulley rotation angle θp exhibits a characteristic with hysteresis, the present embodiment enables accurate ascertainment of the states thereof. Further, the motor control portion 21 switches (changes) driving control of the motor 9 in correspondence with a discrimination result obtained by the discrimination portion 27.

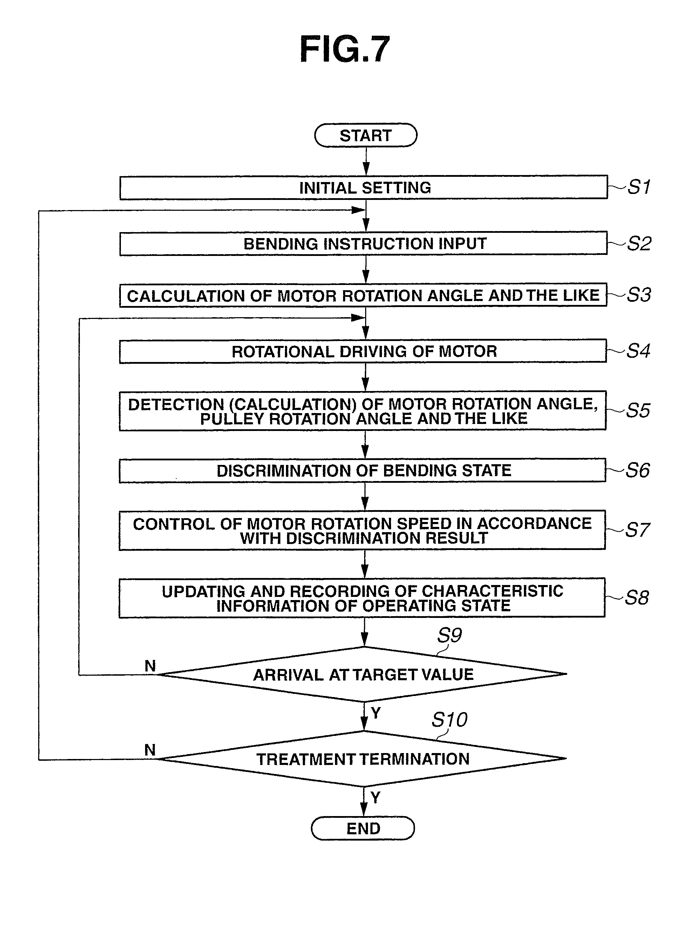

Next, bending control operations according to the present embodiment are described referring to Fig. 7.

When the power of the medical treatment instrument apparatus 1 is turned on and the control apparatus 4 starts to operate, initial setting processing in step S1 starts. In step S1, the control apparatus 4 sets a state in which the shaft portion 7 of the treatment instrument 3 is straight, that is, a neutral state in which the bending portion 10 is not bent, and the motor rotation angles θm in the vertical direction and horizontal direction detected by the encoders 14a and 14b (hereinafter, represented by reference symbol "14") of the motors 9a and 9b and the bending angle 8b of the bending portion 10 are set to 0. Thereafter, the apparatus is placed on standby for input of an instruction.

In step S2, the operator inputs a bending instruction from the input portion 5. Specifically, the operator operates the joystick 36 to tilt the joystick 36 in the desired direction to bend the bending portion 10 and at the desired bending angle.

As shown in step S3, in correspondence with the bending direction and bending angle of the inputted instruction, the motor control portion 21 of the control apparatus 4 refers to the characteristics information of the storage portion 29 at that time, and calculates a rotational direction (rotational drive direction) in which to rotate the motors 9a and 9b (hereinafter, represented by reference symbol "9"), a motor rotation angle θsm, a pulley rotation angle θsp, and a torque value (rotational driving force) Ts.

Note that, although the state at this time is the initial state, input of a bending instruction is performed in an operation state which is different from the initial state depending on the control loop in Fig. 7. In such a case, the rotational direction, the motor rotation angle θsm, the pulley rotation angle θsp and the torque value Ts are calculated by referring to information regarding operation characteristics updated prior to the relevant operation state. The calculated motor rotation angle θsm and torque value Ts serve as instruction values or target values for the motor 9 when driving to bend. Note that, a configuration may also be adopted in which only the motor rotation angle θsm is taken as an instruction value or a target value for the motor 9 when driving to bend.

Next, in step S4, the motor control portion 21 rotationally drives the motor 9 so as to obtain the calculated motor rotation angle θsm.

At this time, as shown in step S5, the encoder 14 and the potentiometers 15a and 15b (hereinafter, represented by reference symbol "15") and the like each detect the rotation angle of the motor 9 and the pulley 13, respectively. Alternatively, the motor rotation angle calculation portion 22 and the pulley rotation angle calculation portion 23 calculate the motor rotation angle and the pulley rotation angle, respectively. Further, the torque calculation portion 24 calculates the torque value.

That is, the control apparatus 4 detects (calculates) the operating states of the motor 9 and the pulley 13. Further, as shown in step S6, the discrimination portion 27 discriminates the bending state.

Subsequently, as shown in step S7, the motor control portion 21 controls the motor rotation speed in accordance with the discrimination result.

In addition, as shown in step S8, the storage portion 29, for example, stores information for the respective operating states of the motor 9 and the pulley 13 in a short fixed cycle, and updates stored characteristics information in accordance with the discrimination result obtained by the discrimination portion 27.

In step S9, the motor control portion 21 discriminates whether or not the motor 9 has been rotated to the target value based on the discrimination result of the discrimination portion 27 or the like into which calculation results of the motor rotation angle calculation portion 22 and the torque calculation portion 24 and the like are inputted.

If the target motor rotation angle θsm has not been reached, the process returns to step S4 to repeat the above described operations. In contrast, if the target value has been reached, in step S 10, the control apparatus 4 judges whether or not the treatment is to be ended. If the treatment is not to be ended, the process returns to step S2 to wait for input of the next bending instruction. In contrast, if the treatment is to be ended, the control apparatus 4 ends the processing shown in Fig. 7.

Fig. 8 shows the details of the processing in step S6 and step S7.

As shown in step S 11, the discrimination portion 27 makes a discrimination with respect to a first bending state (driving force contributing state) Stlla, a second bending state (restoration characteristic state) Stllb, and a third bending state Stllc. More specifically, as the third bending state Stllc, the discrimination portion 27 makes a discrimination with respect to a backlash state Stlld as the number 3-1 bending state and a slackened state Stlle as the number 3-2 bending state.

If the discrimination result in step S11 is the first bending state, as shown in step S12a, the motor control portion 21 performs control that rotates the motor 9 at a normal rotation speed (referred to as "first rotation speed"). After the processing in step S12a, the process advances to step S8.

If the discrimination result in step S11 is the second bending state, as shown in step S12b, the motor control portion 21 performs control that rotates the motor 9 at a rotation speed (driving speed) corresponding to the restoration characteristic state, specifically, control that causes the motor 9 to rotate at a second rotation speed that is a lower speed than the first rotation speed.

In this state, since the bending portion 10 is already caused to rotate by the restoring force, if rotation is performed at the first rotation speed by the motor 9, the speed will become faster than the speed of normal driving for bending. Hence, by rotating at a lower speed than the first rotation speed, driving for bending is performed in a similar operating state in both a case where the restoring force acts and a case where the restoring force does not act. After the processing in step S12b, the process advances to step S8.

Further, if the discrimination result in step S11 is the number 3-1 bending state (backlash state), as shown in step S12c, the motor control portion 21 performs control that rotates the motor 9 at a third rotation speed corresponding to a backlash state, specifically, control that causes the motor 9 to rotate at a third rotation speed that is a higher speed than the first rotation speed.

Furthermore, if the discrimination result in step S11 is the number 3-2 bending state (slackened state), as shown in step S12d, the motor control portion 21 performs control that rotates the motor 9 at a fourth rotation speed that corresponds to a slackened state. More specifically, the motor control portion 21 performs control to rotate the motor 9 at the fourth rotation speed that is a higher speed than the first rotation speed to wind up the wires 11a and 11b so as to remove slackness therefrom. Note that, the third rotation speed and the fourth rotation speed may be set to the same rotation speed.

In the third bending state, the bending portion 10 corresponds to an unresponsive state in which the bending portion 10 is, in effect, not being bent. Therefore, in the third bending state, by making the rotation speed of the motor 9 a high speed, the motor control portion 21 shortens a time period of the unresponsive state and ensures favorable responsiveness and operability.

By controlling the rotation speed of the motor 9 in accordance with the bending state in this manner, favorable operability can be ensured.

Further, after ending the processing in step S 12c, as shown in step S 13 a, the discrimination portion 27 corrects the current characteristics information stored in the storage portion 29 to characteristics information in which the motor rotation angle and the pulley rotation angle at the bending state position at the time that step S12c ends (when the backlash state is eliminated) are associated, and causes the corrected characteristics information to be stored in step S8.

Further, after the processing in step S12d ends, as shown in step S 13b, the discrimination portion 27 corrects the current characteristics information stored in the storage portion 29 to characteristics information in which the motor rotation angle, the pulley rotation angle, and the bending angle at the bending state position at the time that step S12d ends (when the slackened state is eliminated) are associated, and causes the corrected characteristics information to be stored in step S8.

According to the present embodiment that has the above described configuration, a boundary between a range in which it is possible for a restoring force of an elastic member forming the bending portion 10 to contribute to bending of the bending portion 10 and a range in which it is not possible for the restoring force to contribute to bending of the bending portion 10 due to slackness in the wires 11a and 11b can be identified by the identification portion 26.

Further, according to the present embodiment, by utilizing information regarding the boundary identified by the identification portion 26, slackness in the wires 11a and 11b can be detected and characteristics information can be updated in accordance with the slackness, and control can also be performed so as to promptly eliminate an unresponsive state of bending that is caused by the slackness.

Therefore, according to the present embodiment, when using wires in which slackness exists, a bending state that is a case where an unresponsive state occurs in which the bending portion will not be bent can be accurately detected without lowering the responsiveness, and operability when driving to bend the bending portion can be improved.

Furthermore, in the case of driving for bending that exhibits a hysteresis characteristic due to slackness of a wire also, since a configuration is adopted so as to perform a correction with respect to the slackness, a decrease in the accuracy of the control system when driving to bend can be prevented. Note that, to perform rotational control of the motors more simply, a configuration may be adopted in which rotational control is performed taking the above described number 3-1 bending state and number 3-2 bending state collectively as a third bending state.

### (Second Embodiment)

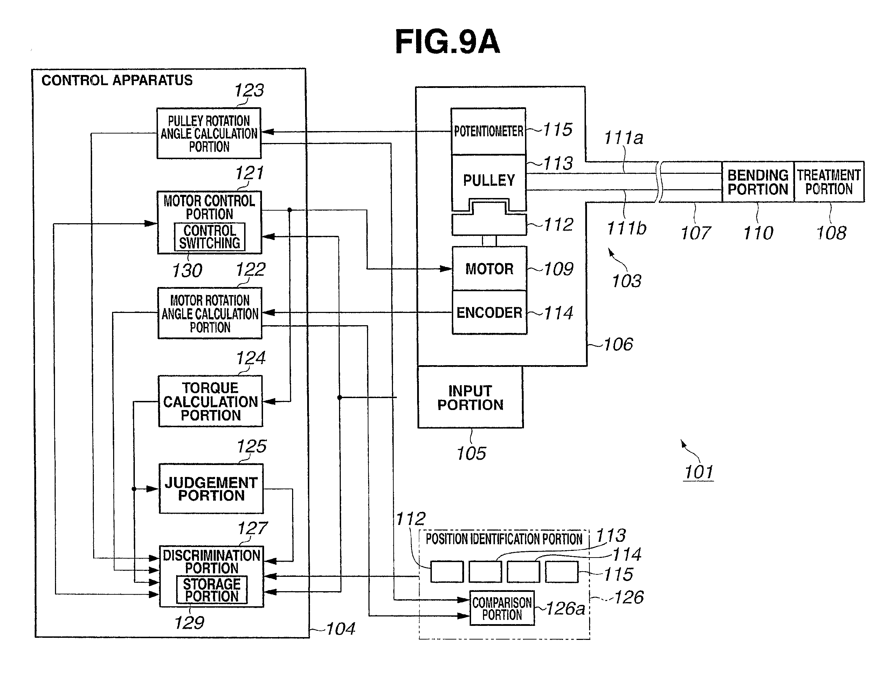

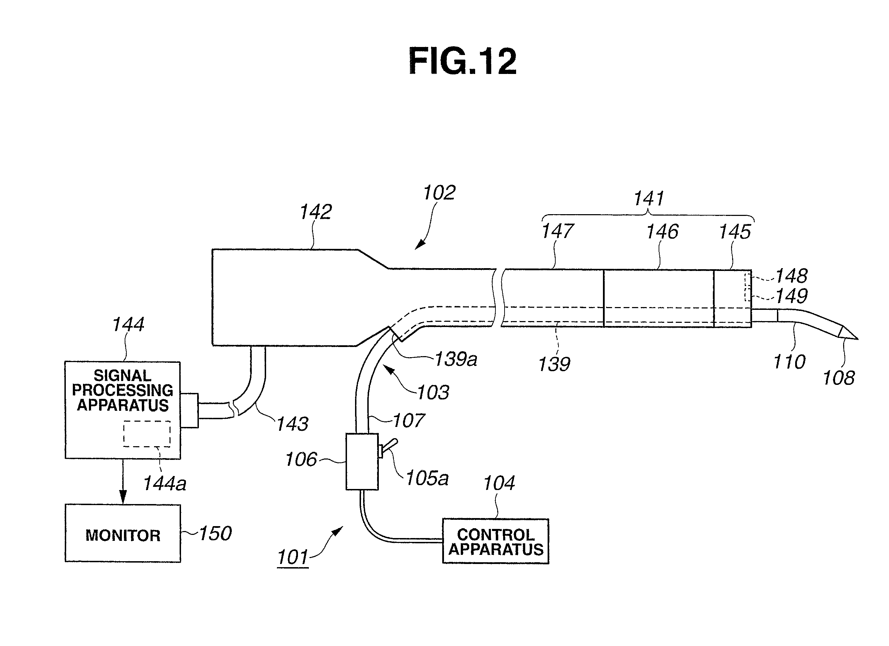

As shown in Fig. 9A, a treatment instrument apparatus 101 according to a second embodiment of a medical control apparatus of the present invention includes, for example, an active treatment instrument (hereunder, referred to simply as "treatment instrument") 103 that actively drives to bend a bending portion 110, that is inserted through the inside of a treatment instrument channel (hereunder, referred to as "channel") 139 of an endoscope 102 as shown in Fig. 12 that is inserted into a body cavity and used.

The treatment instrument apparatus 101 also includes a control apparatus 104 that is connected to the treatment instrument 103 and performs control with respect to the treatment instrument 103, and an input portion 105 that is connected to the control apparatus 104 and with which an operator performs an operation to input instructions. In the example shown in Fig. 12, the input portion 105 is constituted by a joystick apparatus 105a that is provided on a grasping portion 106 at a rear end of the treatment instrument 103. Note that, the joystick apparatus 105a may also be provided at a location other than the grasping portion 106.

The treatment instrument 103 includes a shaft portion 107 as a flexible and elongated insertion portion that is inserted through the inside of the channel 139, a treatment portion 108 that performs treatment and is provided at a distal end of the shaft portion 107, and a motor 109 as a drive portion that is provided at a rear end of the shaft portion 107. Note that, in the specific example shown in Fig. 12, the treatment portion 108 is constituted by, for example, a biopsy needle that extracts a sample of living tissue by puncturing a diseased part or the like. Further, in Fig. 12, the motor 109 that constitutes a drive portion is provided inside the grasping portion 106.

The bending portion 110 that is capable of bending is provided as an active mechanism at a rear end position of the treatment portion 108. The bending portion 110 is connected through a connecting portion 112 and a pulley 113 constituting a pulling portion with a motor 109 that generates a rotational driving force as a driving force through a pair of angle wires (abbreviated as simply "wires") 111a and 111b for causing the bending portion 110 to bend.

By pulling and driving one of the pair of wires 111a and 111b and slackening the other of the pair of wires 111a and 111b in accordance with the torque as a generated rotational driving force, the motor 109 drives the bending of the bending portion 110 toward the side of the wires 11a and 11b. Note that, the wires 111a and 111 b are inserted through the inside of the shaft portion 107 in a slackened state. Further, since the shaft portion 107 is formed in an elongated shape, the bending portion 110 is formed on a distal end side of the shaft portion 107.

The motor 109 includes an encoder 114 as detection means that detects a rotational driving amount (or motor rotation angle) as a driving amount of the motor 109. The encoder 114 is constituted by a rotary encoder or the like.

Further, a potentiometer 115 is attached to the pulley 113 constituting the pulling portion that is rotatably connected through the connecting portion 112 with the motor 109. The potentiometer 115 serves as detection means that detects a pulley rotational driving amount or a pulley rotation angle as a pulling portion driving amount of the pulley 113. Note that, a configuration may also be adopted that detects a pulley rotational driving amount or a pulley rotation angle using a rotary encoder instead of the potentiometer 115.

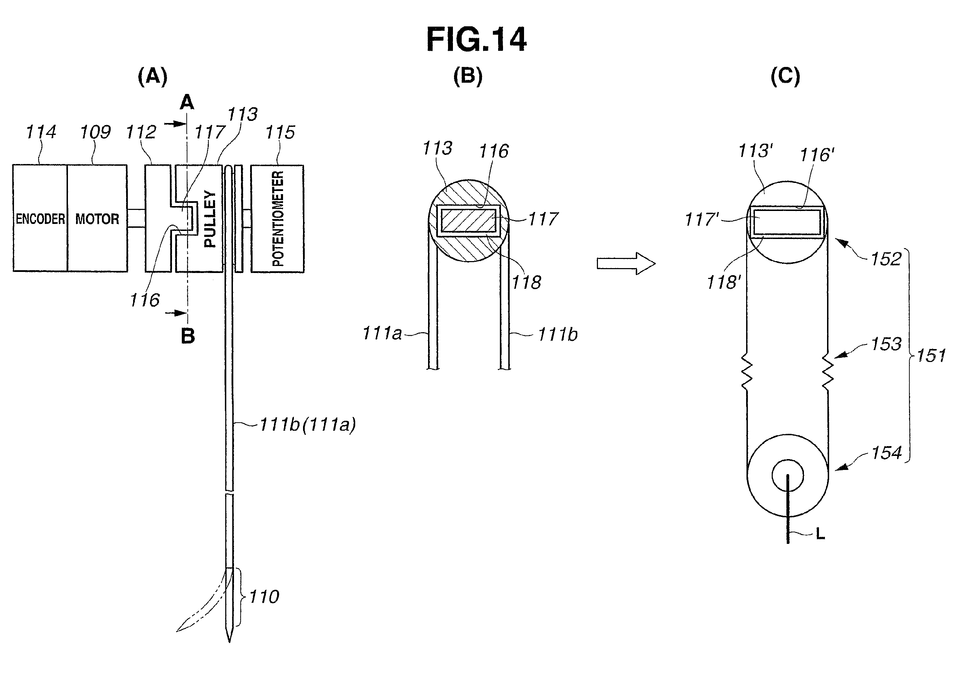

The connecting portion 112 that connects the motor 109 and the pulley 113 without an intervening wire includes, as shown in Fig. 14(A) and Fig. 14(B), a rectangular concave portion 116 that is provided (integrally with the pulley 113) in the center of the disk-shaped pulley 113, and a convex portion 117 that is connected to a rotation shaft of the motor 109 and is engagingly inserted into the concave portion 116.

The connecting portion 112 also includes a backlash portion (or play portion) 118 that generates a positional relationship which cannot transmit (does not transmit) a rotational driving force from the motor 109 between the concave portion 116 and the convex portion 117. Note that, Fig. 14(B) shows a cross-sectional view along a line A-B in Fig. 14(A).

The connecting portion 112 has a positional relationship that transmits a rotational driving force (torque) from the motor 109 as a result of contact between an engagement surface of an inner face of the concave portion 116 and an engagement surface of a side face of the convex portion 117, and also enters a state of a positional relationship that cannot transmit the rotational driving force in a state in which the two engagement surfaces do not contact against each other by means of the backlash portion 118.

In the present embodiment, the connecting portion 112 is configured to directly connect the motor 109 and the pulley 113 within a short distance (more specifically, approximately coaxially) without an intervening wire (which is long and with which a time delay arises when transmitting a driving force), and thus a decline in responsiveness is prevented.

Note that, the present invention is not limited to a configuration in which the concave portion 116 is integrally provided in the pulley 113. A configuration may also be adopted in which the concave portion 116 is provided on the motor 109 side, and the convex portion 117 is provided on the pulley 113 side.

As shown in Fig. 9A, the control apparatus 104 includes a motor control portion 121 that has a function that drives the motor 109, and a motor rotation angle calculation portion 122 that calculates a motor rotation angle based on a detection signal with respect to a rotational driving amount or a rotation angle of the motor 109 that is outputted from the encoder 114.

The control apparatus 104 also includes a pulley rotation angle calculation portion 123 that calculates a pulley rotation angle based on a detection signal with respect to a pulley rotation angle from the potentiometer 115, and a torque calculation portion 124 that calculates or detects a torque of the motor 109 based on a motor drive current and a drive voltage when the motor 109 is driven by means of a motor drive signal.

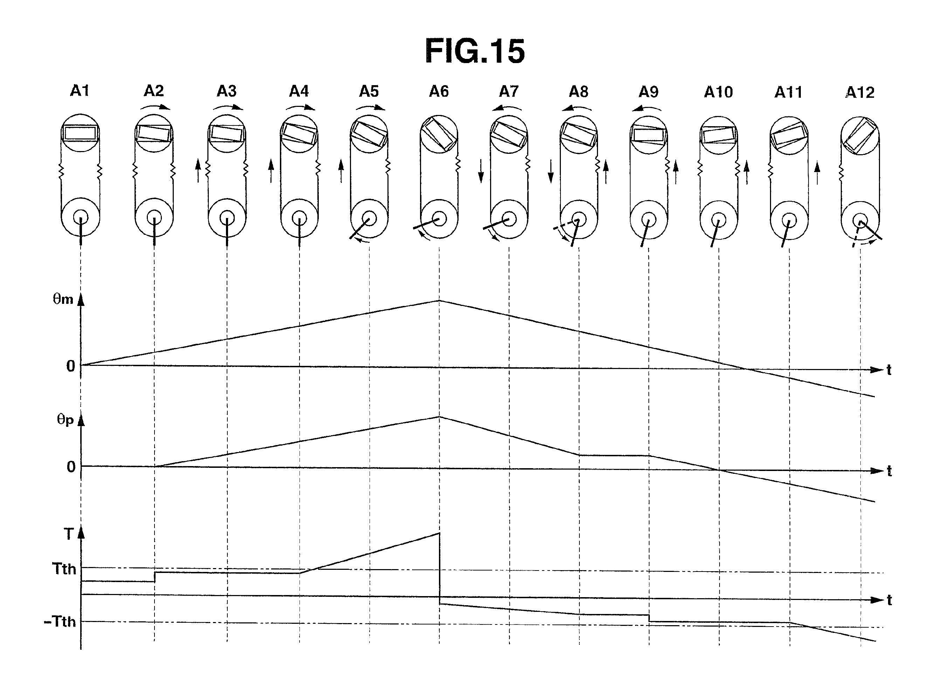

The control apparatus 104 further includes a judgment portion 125 that, based on the torque from the torque calculation portion 124, judges whether or not the treatment instrument apparatus 101 is in a driving force contributing state in which a rotational driving force of the motor 109 as a drive portion contributes to driving to bend the bending portion 110. The judgment portion 125 makes a comparison to determine whether or not (an absolute value of) a torque value T from the torque calculation portion 124 is equal to or greater than a torque threshold value Tth that is used for judgment (see Fig. 15). If |T|≥Tth, the judgment portion 125 judges that the treatment instrument apparatus 101 is in the driving force contributing state, while if |T|<Tth, the judgment portion 125 judges that the treatment instrument apparatus 101 is not in the driving force contributing state.

Furthermore, the control apparatus 104 includes a discrimination portion 127 that discriminates three bending states, described later, based on a judgment result of the judgment portion 125 and a detection result of a position identification portion 126 that detects a specific bending state position and the like. Note that, in practice, the judgment portion 125 judges the driving force contributing state as being a first bending state of the three bending states.

The position identification portion 126 detects a specific bending state position at which the bending portion 110 maintains a specific bending state without a torque that drives to bend the bending portion 110 being applied (generated) by the motor 109. Specifically, the bending portion 110 is bent as far as a specific bending state position by a restoring force produced by an elastic member forming the bending portion 110 that is described later, without the motor 109 applying (generating) a torque that drives to bend the bending portion 110. The position identification portion 126 detects (identifies) the specific bending state position.

As shown in Fig. 9A, the position identification portion 126 has a configuration that includes the connecting portion 112, the pulley 113 as a pulling portion, the encoder 114 as a driving amount detection portion, the potentiometer 115 as a pulling portion driving amount detection portion, and a comparison portion 126a that are illustrated in Fig. 9A. In addition to this definition of the configuration of the position identification portion 126, the position identification portion 126 may also be defined as a configuration that includes the comparison portion 126a into which a change amount of a motor rotation angle and a change amount of a pulley rotation angle are inputted.

The comparison portion 126a detects a specific bending state position at which the bending portion 110 is in a specific bending state by performing a comparison with respect to a change amount of a motor rotation angle detected by the encoder 114 or calculated by the motor rotation angle calculation portion 122 and a change amount of a pulley rotation angle detected by the potentiometer or calculated by the pulley rotation angle calculation portion, or by a comparison with a threshold value.

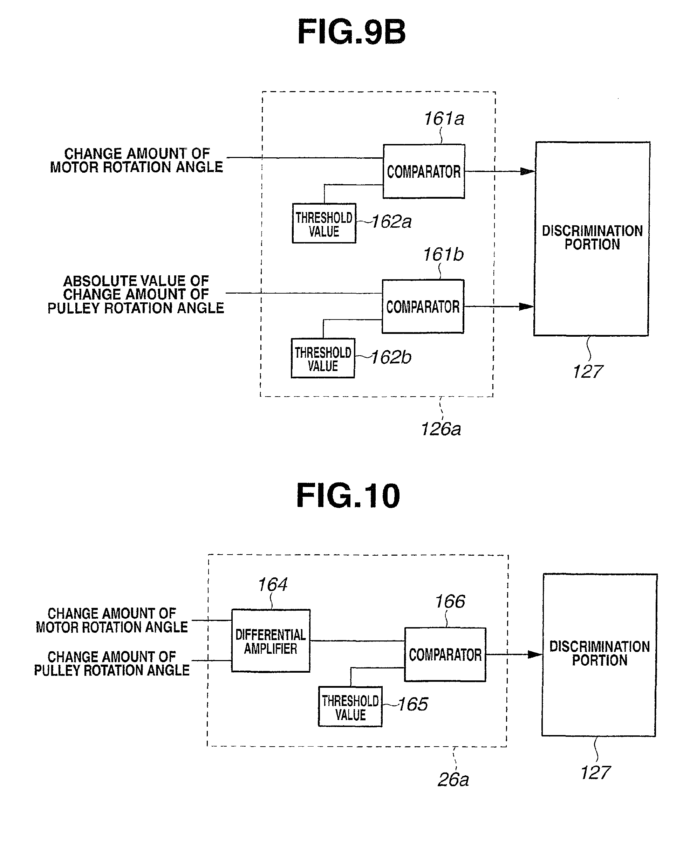

Fig. 9B illustrates a configuration example of the comparison portion 126a.

The comparison portion 126a includes a first comparator 161a and a window-type second comparator 161b. The comparison portion 126a outputs a comparison result of the first comparator 161a and the second comparator 161b to the discrimination portion 127.

A change amount of a motor rotation angle and a first threshold value 162a that is used for judging the change amount of the motor rotation angle are inputted to the first comparator 161a. The first comparator 161a outputs a result of comparison thereof to the discrimination portion 127.

Further, an absolute value of a change amount of the pulley rotation angle, and a second threshold value 162b that is used for judging the absolute value of the change amount are inputted to the second comparator 161b. The second comparator 161b outputs a result of comparison thereof to the discrimination portion 127. For example, the second comparator 161b outputs a positive comparison result if the absolute value of the change amount of the pulley rotation angle is within the second threshold value 162b, and outputs a negative comparison result if the absolute value of the change amount is outside the second threshold value 162b. Note that, a circuit that calculates an absolute value of a change amount of the pulley rotation angle based on the change amount is provided at a stage prior to the comparator 161b (not shown).

A change amount of the motor rotation angle is calculated based on a motor rotation angle that changed per short predetermined time period ta. A change amount of the pulley rotation angle is also calculated in a similar manner based on a pulley rotation angle that changed per time period ta, by sampling (extracting) pulley rotation angles at cycles of the short predetermined time period ta.

The first threshold value 162a, for example, is set to a value that corresponds to a change of a motor rotation angle θm as shown in Fig. 15 that is described later. Note that, Fig. 15 shows a motor rotation angle θm and a pulley rotation angle θp and the like that correspond to a bending angle of the bending portion 110 in a case where the bending portion 110 has been bent.

More specifically, the first threshold value 162a is set in correspondence with a change characteristic of the motor rotation angle θm that corresponds to a state from reference symbol A6 to A8 in Fig. 15. In this case, the first threshold value 162a is set in correspondence with a characteristic that the motor rotation angle θm decreases over time, and is set to a value that includes an allowable margin from the characteristic shown in Fig. 15.

In other words, even if the motor rotation angle θm changes so as to decrease with a characteristic that deviates to some extent from the characteristic shown in Fig. 15, the first comparator 161a outputs a comparison result (for example, an output signal with positive polarity) to the effect that the motor rotation angle θm is changing by a change amount that is within a permitted range.

The second threshold value 162b, for example, is set to a value that corresponds to a change in the pulley rotation angle θp shown in reference symbol A8 to A9 in Fig. 15. In this case, to enable judgment of a state in which the pulley rotation angle θp does not change and maintains a constant value as shown by the state between reference symbols A8 and A9, the second threshold value 162b, for example, is set to a positive value that is close to 0 (that is less than an absolute value of a negative change amount in the case of the state between reference symbols A7 and A8).

The second comparator 161b judges whether or not an absolute value of a change amount in the pulley rotation angle is within the second threshold value 162b.

That is, the second comparator 161b is a window-type comparator as described above, and detects whether or not the absolute value of the change amount of the pulley rotation angle is a value close to 0 by performing a comparison with the second threshold value 162b. The second comparator 161b, for example, outputs a positive output signal when the absolute value of the change amount of the pulley rotation angle is within the second threshold value 162b, and outputs a negative output signal in other cases (when the absolute value is not within the second threshold value 162b).

Therefore, the second comparator 161b outputs an output signal with negative polarity in a case of the state from reference symbol A7 to immediately before A8, and outputs an output signal with positive polarity from the state denoted by reference symbol A8 onwards. Based on the comparison outputs of the first comparator 161a and the second comparator 161b, the discrimination portion 127 identifies (discriminates) that the bending portion 110 has been bent to the specific bending state position by the restoring force when the output signal of the first comparator 161a is positive and the output signal of the second comparator 161b has changed from negative to positive.

Based on the comparison outputs of the comparison portion 126a received through the discrimination portion 127, or without passing through the discrimination portion 127, the motor control portion 121 performs control that changes (switches) the control method of the motor 109.

Thus, the comparison portion 126a included in the position identification portion 126 detects a specific bending state position.

Note that, a state in which a state where a change amount of the pulley rotation angle is 0 continues from reference symbol A8 to reference symbol A9 in Fig. 15 corresponds to a bending state in which the motor 109 and the pulley 113 do not engage due to the backlash portion 118, that is, a backlash state.

When a state from reference symbol A1 to A2 in which the pulley rotation angle θp is a value that does not change in Fig. 15 is also included in the aforementioned backlash state, the comparison portion 126a can also detect the backlash state from reference symbol A1 to A2 by, in a case where the motor rotation angle changes, utilizing a comparison result of the second comparator 161b such that a change amount of the pulley driving amount is within the threshold value 162b that is close to 0.

However, in this case, it is sufficient to adopt a configuration on the first comparator 161 a side that also detects a case where a change amount of the motor rotation angle is a positive value. For example, in Fig. 9B, although the comparator 161a and the threshold value 162a correspond to a configuration that detects a case where the change amount of the motor rotation angle is a negative amount, it is good to adopt a configuration (not shown) in which a comparator and a threshold value are further added that corresponds to detection of a case where the change amount of the motor rotation angle is a positive value and in which the output of the comparator is inputted to the discrimination portion 127.

A configuration of a modification shown in Fig. 10 may be adopted instead of the configuration shown in Fig. 9B as a configuration that detects a specific bending state position. This modification is also a configuration example for identifying a state from reference symbol A8 to A9 in Fig. 15. The comparison portion 126a includes a differential amplifier 164 that compares a change amount of the motor rotation angle and a change amount of the pulley rotation angle and outputs a difference value, and a comparator 166 that compares the difference value and the threshold value 165. An output signal of the comparator 166 is outputted to the discrimination portion 127.

The differential amplifier 164 outputs a first difference value as a difference value between a change amount of the motor rotation angle and a change amount of the pulley rotation angle in the case of a state from reference symbol A7 to immediately before reference symbol A8, and the difference value becomes a second difference value that is only a change amount of the motor rotation angle from reference symbol A8 onwards (up to S9).

Therefore, the threshold value 165 for enabling judgment with respect to a case in which there is only a change amount of the motor rotation angle is, for example, set to an intermediate value between the first difference value and the second difference value, and a comparison result is inverted with respect to a case where an output signal of the differential amplifier 164 that is inputted to the comparator 166 is the first difference value and a case where the aforementioned output signal is the second difference value.

When the bending portion 110 is bent in the specified position state, the comparison portion 126a detects the state as a result of the polarity of the output signal of the comparator 166 being inverted. Accordingly, when the polarity of the output signal of the comparator 166 is inverted, the discrimination portion 127 discriminates that the bending portion 110 is bent in the specified position state.

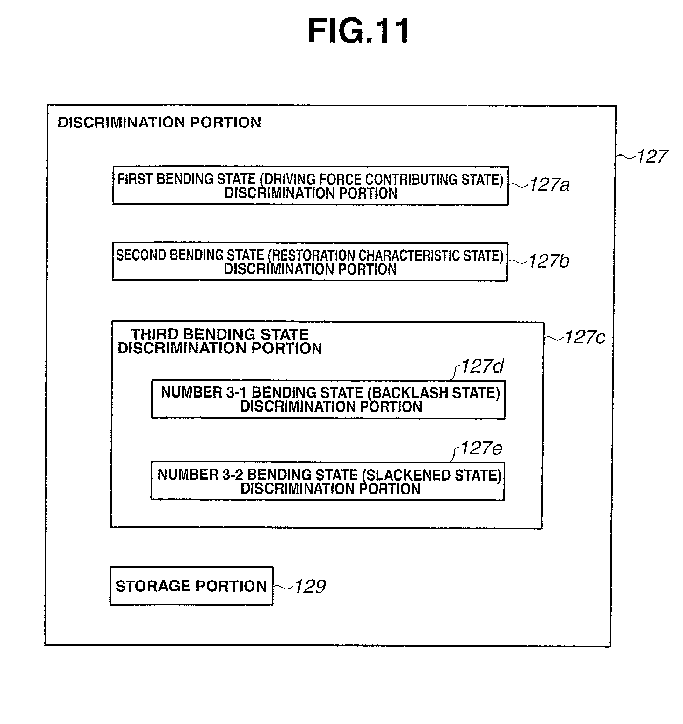

As shown in Fig. 11, the judgment portion 127 includes first bending state discrimination portion 127a that discriminates the above described driving force contributing state as a first bending state based on a judgment result obtained by the judgment portion 125. The judgment portion 127 also discriminates bending states that are not the driving force contributing state as two bending states, that is, a second bending state and a third bending state, respectively.

The discrimination portion 127 includes a second bending state discrimination portion 127b that, based on a judgment result from the judgment portion 125, discriminates a bending state in which, immediately after changing to a bending state that is not the driving force contributing state from the driving force contributing state, the bending angle of the bending portion 110 changes as far as the aforementioned specific bending state position when the pulley 113 is rotated without the motor 109 applying a torque to the load side (pulley side) as being the second bending state.

The second bending state corresponds to a state in which the bending portion 110 is bent (until reaching the specific bending state position) by a restoring force produced by the elastic member forming the bending portion 110 in a state in which a torque that bends the bending portion 110 is not generated by the motor 109, when an instruction to perform a bending operation to return the bending portion 110 to the neutral state side is made immediately after the bending portion 110 was bent by a large amount. Therefore, a function that discriminates a second bending state by means of the discrimination portion 127 corresponds to a function that discriminates a restoration characteristic state (as a characteristic state in which bending is caused by a restoring force).

A state where the bending angle of the bending portion 110 changes in the second bending state is one in which the pulley rotation angle of the pulley 113 changes in a decreasing direction, and hence the discrimination portion 127 discriminates the second bending state based on that change characteristic. Further, the second bending state is maintained until the specific bending state position (denoted by reference symbol A8 in Fig. 15) at which the restoring force becomes 0 (or a state where the restoring force balances with frictional forces or the like).

The discrimination portion 127 also includes a third bending state discrimination portion 127c (see Fig. 11) that discriminates, as a third bending state, a bending state in which a torque value T generated by the motor 109 is not detected as a value that is equal to or greater than a (torque threshold value Tth as a) predetermined value, in a bending state that is not the driving force contributing state excluding the aforementioned second bending state.

The third bending state corresponds to a state where the motor 109 and the pulley 113 re-engage (one engagement is disconnected and another engagement is made) by means of the backlash portion 118 that corresponds to a bending state at the aforementioned specific bending state position, or a state where slackness has arisen in the wires 111a and 111b.

In addition, the discrimination portion 127 includes a number 3-1 bending state discrimination portion 127d and a number 3-2 bending state discrimination portion 127e (see Fig. 11) that further discriminate the third bending state into two bending states, namely, a number 3-1 bending state and a number 3-2 bending state.

That is, the discrimination portion 127 discriminates the number 3-1 bending state in which the pulley rotation angle does not change with respect to a change amount of the motor rotation angle, and the number 3-2 bending state in which the pulley rotation angle also changes with respect to a change amount of the motor rotation angle. Note that, the number 3-1 bending state and the number 3-2 bending state may also be defined as a third bending state and a fourth bending state, respectively.

The aforementioned number 3-1 bending state corresponds to a state in which the motor 109 and the pulley 113 re-engage by means of the backlash portion 118, in other words, a state (backlash state) in which the motor 109 and the pulley 113 are not engaged in the bending direction. The number 3-2 bending state corresponds to a state in which slackness of the wires 111a and 111b has arisen. That is, the discrimination portion 127 has a function of a backlash state judgment portion that judges a backlash state as the number 3-1 bending state discrimination portion 127d, and a function of a slackened state judgment portion that judges a slackened state of the wires as the number 3-2 bending state discrimination portion 127e.

As shown in Fig. 11, the discrimination portion 127 includes the first bending state discrimination portion 127a, the second bending state discrimination portion 127b and the third bending state discrimination portion 127c.

Note that, a boundary between the second bending state and the number 3-1 bending state in the third bending shape is the aforementioned specific bending state position, and both bending states can be discriminated based on information regarding the specific bending state position.

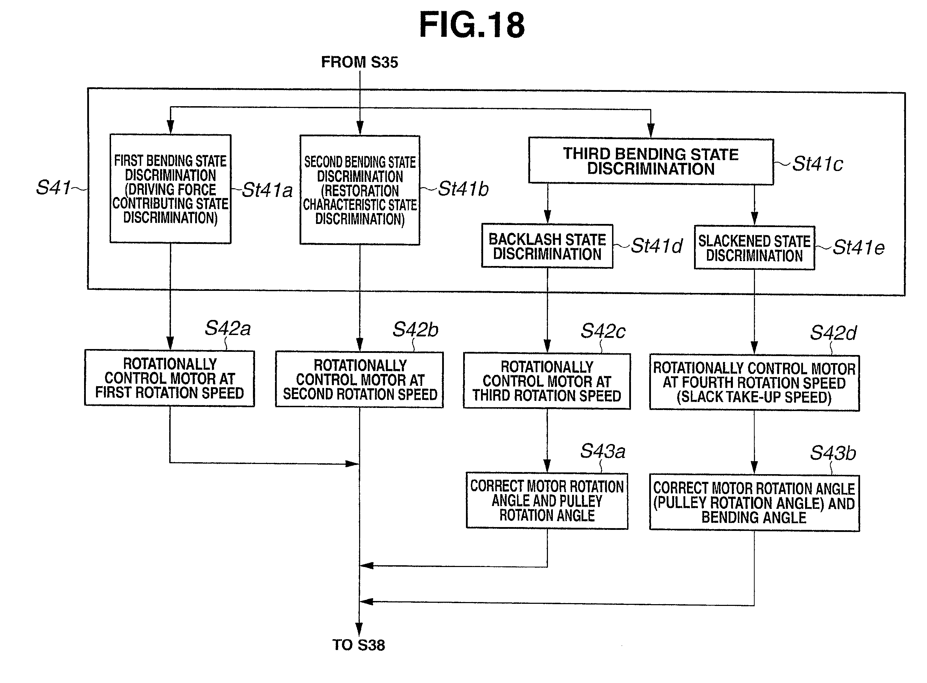

The discrimination portion 127 sends the discrimination result to the motor control portion 121. The motor control portion 121 controls so as to perform rotational driving that switches the rotation speed (as driving speed) of the motor 109 in accordance with the discrimination result (specifically, controls as shown in Fig. 18 that is described later).

In addition, the discrimination portion 127 incorporates a storage portion 129 that memorizes (stores) respective pieces of information regarding a bending angle of the bending portion 110, a motor rotation angle of the motor 109 and a pulley rotation angle of the pulley 113 as mutually-associated characteristics information. The discrimination portion 127 refers to the characteristics information of the storage portion 129 as necessary. Note that, the storage portion 129 may also be provided outside the discrimination portion 1127.

The storage portion 129 is connected to the motor control portion 121, the motor rotation angle calculation portion 122, the pulley rotation angle calculation portion 123, the torque calculation portion 124, the judgment portion 125, the position identification portion 126, the input portion 105 and the like.

In addition to storing the above described characteristics information, the storage portion 129 chronologically (on a time-series basis) stores information such as a bending angle of the bending portion 110, a motor rotation angle of the motor 109, a pulley rotation angle of the pulley 113, a torque, a judgment result obtained by the judgment portion 125, a detection result of the position identification portion 126, information relating to control switching by the motor control portion 121, a bending instruction value from the input portion 105 and the like.

Note that, information regarding a bending angle of the bending portion 110 is previously associated with information regarding a pulley rotation angle or a motor rotation angle and stored in the storage portion 129, and the information regarding a bending angle of the bending portion 110 is also updated in a time-series manner based on the information in time series regarding the pulley rotation angle or the motor rotation angle.

In response to input of an instruction to bend the bending portion 110 from the input portion 105, the motor control portion 121 performs control to apply (supply) a motor drive signal to the motor 109 to rotationally drive the motor 109. When performing control to rotationally drive the motor 109, the motor control portion 121 performs control that rotationally drives the motor 109 by switching the driving speed (motor rotation speed) of the motor 109 in accordance with the three bending states (four bending states when the fact that the third bending state is split into two states is taken into consideration) based on the discrimination result obtained by the discrimination portion 127. Accordingly, the motor control portion 121 includes a control switching portion 130 that performs control for switching the rotation speed according to the three bending states.

Note that, an instruction that is inputted from the input portion 105 is also inputted to the storage portion 129 inside the discrimination portion 127 as described above, and information regarding the inputted instruction is also stored on a time-series basis in the storage portion 129.

Further, when the motor control portion 121 performs control that rotationally drives the motor 109 by means of a motor drive signal based on the discrimination result obtained by the discrimination portion 127, the storage portion 129 performs a correction operation if correction (updating) of characteristics information is required, and updates the characteristics information (under control by the discrimination portion 127). Note that, a configuration may also be adopted in which correction and updating of characteristics information of the storage portion 129 is performed under control of the motor control portion 121 instead of being performed under control of the discrimination portion 127.

Specifically, according to the discrimination result obtained by the discrimination portion 127, in a driving state in which slackness occurs in the wires 111a and 111b, to remove the slackness in a short time, the motor control portion 121 outputs a motor drive signal to the motor 109 to cause the motor 109 to rapidly rotate so as to take up the slackness in the wire 111 by means of the pulley 113, and also corrects and updates information regarding the motor rotation angle of the motor 109 and the pulley rotation angle of the pulley 113 with respect to the bending angle of the bending portion 110 by an amount corresponding to the amount of the slackness.

By correcting the information in this manner, even in a case in which slackness has arisen in the wire 111, characteristics information regarding the bending angle of the bending portion 110 and the motor rotation angle and pulley rotation angle can be maintained so as to match the actual driving state (operating state). Furthermore, in a case where bending has been repeated also, the bending portion 110 can be subjected to accurate bending control so as to enter a state of a bending angle that has been instructed by an input operation from the input portion 105. Further, in a case where a backlash occurs and the backlash is eliminated, the storage portion 129 updates the characteristics information with respect to the motor rotation angle and the pulley rotation angle at the time point that the backlash is eliminated.

Thus, in a case where hysteresis has occurred also, a configuration is adopted such that the characteristics information is updated to characteristics information that corresponds to the hysteresis, and accurate and favorable bending control can be performed.

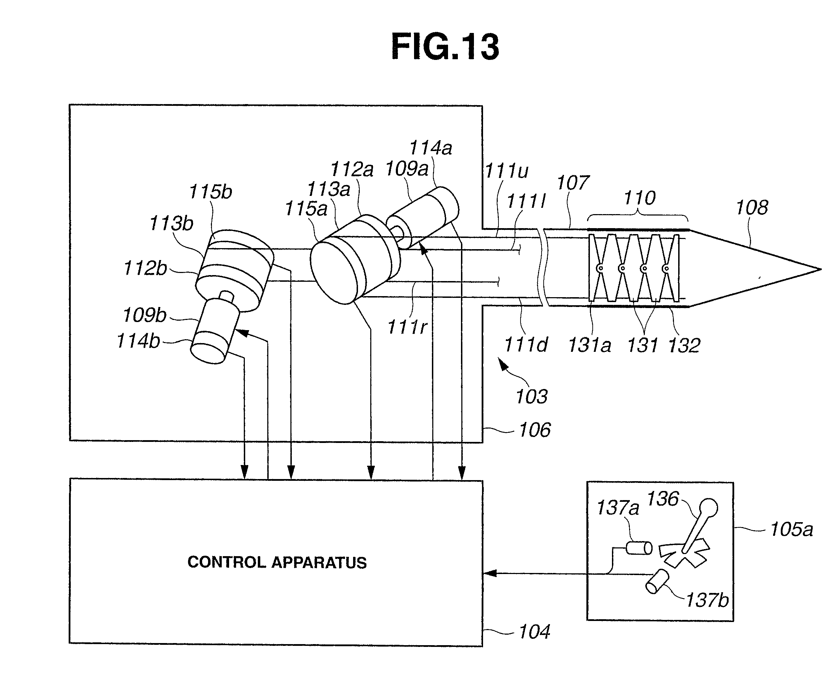

Fig. 13 illustrates a specific configuration example of the treatment instrument 103. As shown in Fig. 13, a biopsy needle that utilizes puncturing is formed as the treatment portion 108 at the distal end of the shaft portion 107. The bending portion 110 is formed at the rear end of the biopsy needle. In the bending portion 110, a plurality of bending pieces 131 are provided that have a substantially annular shape. Portions of the bending pieces 131 that are adjacent to each other in the longitudinal direction of the shaft portion 107 are pivotably connected by rivet portions 131a.

The bending direction of each bending piece 131 is determined by the position at which the rivet 131a is provided. The rivets 131a are disposed at horizontal and vertical positions in an alternating manner or at appropriate cycles, enabling the bending pieces 131 to bend in the vertical and horizontal directions.

Note that, Fig. 13 is a simplified view showing only the rivets 131a that cause bending in the vertical direction. Furthermore, wires 111u, 111d and 111l, 111r for bending in the vertical direction and the horizontal direction are inserted through the shaft portion 107, and the distal ends of the wires 111u, 111d and 111l, 111r are fixed to the treatment portion 108.

Furthermore, the rear ends of the wires 111u, 111d and 111l, 111r are looped over a vertical bending pulley 113a and a horizontal bending pulley 113b that are disposed inside the grasping portion 106 whose diameter is extended at the rear end of the shaft portion 107.

The pulleys 113a and 113b are connected to the rotation shafts of motors 109a and 109b, respectively, through connecting portions 112a and 112b in which the above described backlash portion is provided. The motors 109a and 109b are freely rotated forward or backward according to a motor drive signal from the motor control portion 121.

Concurrently with the rotations of the motors 109a and 109b, the respective pulleys 113a and 113b that are connected through the connecting portions 112a and 112b in which the backlash portion is provided also rotate, and the wires 111u, 111d and 1111, 111r that are respectively looped over the pulleys 113a and 113b are pulled and slackened, respectively. Thus, the bending portion 110 is driven to bend in the direction of the pulled wire.

In addition, encoders 114a and 114b are connected to the rotation shafts of the motors 109a and 109b, respectively, and potentiometers 115a and 115b are connected to the pulleys 113a and 113b, respectively.

Note that, the outer circumferential sides of the aforementioned bending pieces 131 are covered by an outer sheath member formed by a bending rubber tube 132 as an elastic member having an elastic characteristic that seals and protects the bending portion 110 in a freely bendable manner. By means of elastic force of the outer sheath member forming the bending portion 110 and the like, in a case where the bending portion 110 is bent to a particularly large degree, a restoring force arises that attempts to return the bending portion 110 to a neutral state in which the bending portion 110 does not bend.

Further, the joystick apparatus 105a that, for example, constitutes the input portion 105 includes a joystick 136 that can be tilted arbitrarily in the vertical and horizontal directions, respectively, and encoders 137a and 137b that detect tilting angles of the joystick 136 in the vertical and horizontal directions, respectively.

The direction that the joystick 136 is tilted in is the bending instruction direction with respect to the bending portion 110, and the tilting angle is the instruction value for the bending angle of the bending portion 110.

Detection signals from the encoders 137a and 137b are inputted to, for example, the motor control portion 121 in the control apparatus 104. That is, the bending instruction direction and the instruction value for the bending angle are inputted to the motor control portion 121 from the joystick apparatus 105a as bending instruction input means.

The motor control portion 121 refers to characteristics information stored in the storage portion 129 and the like to determine motor rotation angles of the motors 109a and 109b with respect to the instruction value, and rotationally drives the motors 109a and 109b so that the rotation angles of the motors 109a and 109b detected by the encoders 114a and 114b follow the instruction value.

In practice, since slackness arises in the wires 111a and 111b, in the present embodiment, bending states in which slackness or the like is present/absent are discriminated by the discrimination portion 127.

As shown in Fig. 12, the endoscope 102 includes an insertion portion 141 that is inserted into a body cavity, an operation portion 142 provided at a rear end of the insertion portion 141, and a universal cable 143 that is extended from the operation portion 142. An end portion of the universal cable 143 is detachably connected to a signal processing apparatus 144.

The insertion portion 141 of the endoscope 102 includes a distal end portion 145 provided at a distal end of the insertion portion 141, a freely bendable bending portion 146 provided at a rear end of the distal end portion 145, and a flexible portion 147 that has flexibility and extends from the rear end of the bending portion 146 to the front end of the operation portion 142.

An illuminating window 148 that emits illuminating light and an observation window 149 that is formed adjacent to the illuminating window 148 are provided in the distal end portion 145 of the insertion portion 141. Further, the channel 139 through which a treatment instrument can be inserted is provided in the insertion portion 141. A rear end of the channel 139 opens as a treatment instrument insertion port 139a in the vicinity of the front end of the operation portion 142. The operator such as a surgeon can insert the treatment instrument 103 from the treatment instrument insertion port 139a to perform treatment under observation with the endoscope 102.

Further, the signal processing apparatus 144 incorporates a signal processing circuit 144a that generates a video signal based on a signal that is picked up by an unshown objective lens disposed in the observation window 149 and an image pickup device disposed at an image formation position thereof. A video signal generated by the signal processing circuit 144a is outputted to a monitor 150 as a display apparatus. A picked-up image that has been picked up by the image pickup device is displayed as an endoscopic image on a display surface of the monitor 150.

In the present embodiment, the bending portion 110, the pulley 113 that is rotatably suspended via the bending portion 110 and the wires 111a and 111b, and the motor 109 connected through the connecting portion 112 with the pulley 113 that are shown in Fig. 14(A) and Fig. 14(B), are illustrated more simply by a schematized model 151 shown in Fig. 14(C).

Note that, the wires 111a and 111b shown in Fig. 14 represent the wires 111u and 111d or the wires 1111 and 111r in Fig. 13. Furthermore, the motor 109 in Fig. 14 corresponds to the motor 109a or 109b in Fig. 13, the pulley 113 in Fig. 14 corresponds to the pulley 113a or 113b in Fig. 13, and the connecting portion 112 in Fig. 14 corresponds to the connecting portion 112a or 112b in Fig. 13.

In the model 151 shown in Fig. 14(C), the connecting portion 112 between the motor 109 and the pulley 113 of Fig. 14(B) is represented by a connecting portion model 152 that has a backlash, the wires 111a and 111b that have slackness are represented by a wire model 153 in which slackness is schematized by a spring, and the bending portion 110 to which the distal ends of the wires 111a and 111b are attached is represented by a bending portion model 154 with concentric circles.

In the connecting portion model 152, the pulley 113 in Fig. 14(B) is represented by a circular pulley model 113', the concave portion 116 of the pulley 113 is represented by a concave portion model 116', the convex portion 117 of the connecting portion 112 is represented by a rectangular convex portion model 117', and the backlash portion 118 is represented by a backlash portion model 118'.

Further, slackness in the wires 111a and 111b in Fig. 14(A) and Fig. 14(B) is represented by schematized wire models 111a' and 111b' (wire models 111a' and 111b' are represented by the wire model 153) that are represented by spring patterns. In the wire model 153, a wire state of a portion without slackness is shown by a linear wire model, and a wire state of a portion with slackness is shown by a wire model that has a spring pattern.

Further, in the bending portion model 154 with concentric circles which models a bending state of the bending portion 110, a bending state of the bending portion 110 is represented in virtual form by a bending direction line L. For example, in a state in which the bending direction line L extends downward in a straight line from the concentric circles, the bending portion 110 is in a neutral state in which the bending portion 110 does not bend in the vertical direction (or horizontal direction). Note that, in Fig. 14(A), the bending portion 110 that is illustrated using a solid line indicates a straight state, while the bending portion 110 that is illustrated using a chain double-dashed line indicates a bent state.

The treatment instrument apparatus 101 of the present embodiment as a medical control apparatus according to the above described configuration includes: the shaft portion 107 as an insertion portion that includes the bending portion 110 on a distal end side; the motor 109 as a drive portion that generates a driving force for subjecting the bending portion 110 to a bending operating (driving for bending); the wires 111a and 111b that extend from the bending portion 110 and can be pulled by the drive portion, and that are connected with slackness to the bending portion 110; the judgment portion 125 that judges whether or not a driving force of the drive portion is in a driving force contributing state that contributes to driving to bend the bending portion 110; the torque calculation portion 124 as a driving force detection portion that detects a driving force generated at the drive portion; the position identification portion 126 that detects a specific bending state position at which the bending portion 110 maintains a specific bending state without application of a driving force that drives to bend the bending portion 110 by the drive portion; and the discrimination portion 127 that discriminates, as bending states of the bending portion 110, a first bending state that is the driving force contributing state, a second bending state in which a bending state of the bending portion 110 is changed as far as the specific bending state position immediately after the state is changed from the driving force contributing state to a bending state that is not the driving force contributing state by the drive portion, and a third bending state in which a driving force generated by the drive portion is not detected as a value that is equal to or greater than a predetermined value in a bending state that is not the driving force contributing state excluding the second bending state.

Next, operations of the treatment instrument apparatus 101 of the present embodiment are described.

In Fig. 15, reference symbols A1 to A12 denote representative bending states when performing operations in which the motor 109 shown in Fig. 14 is rotationally driven to rotate the bending portion 110 by a predetermined angle via the pulley 113 that is connected to the motor 109 through the connecting portion 112, and thereafter the bending portion 110 is rotated by an appropriate angle in the opposite direction.

Note that, in Fig. 15, representative bending states when the bending portion 110 is driven to bend using the model shown in Fig. 14 are denoted by reference symbols A1 to A12 in the uppermost section, the corresponding motor rotation angles θm are shown in the second section, pulley rotation angles θp are shown in the third section, and torque values T of the motor 109 that are calculated by the torque calculation portion 124 are shown in the fourth section.

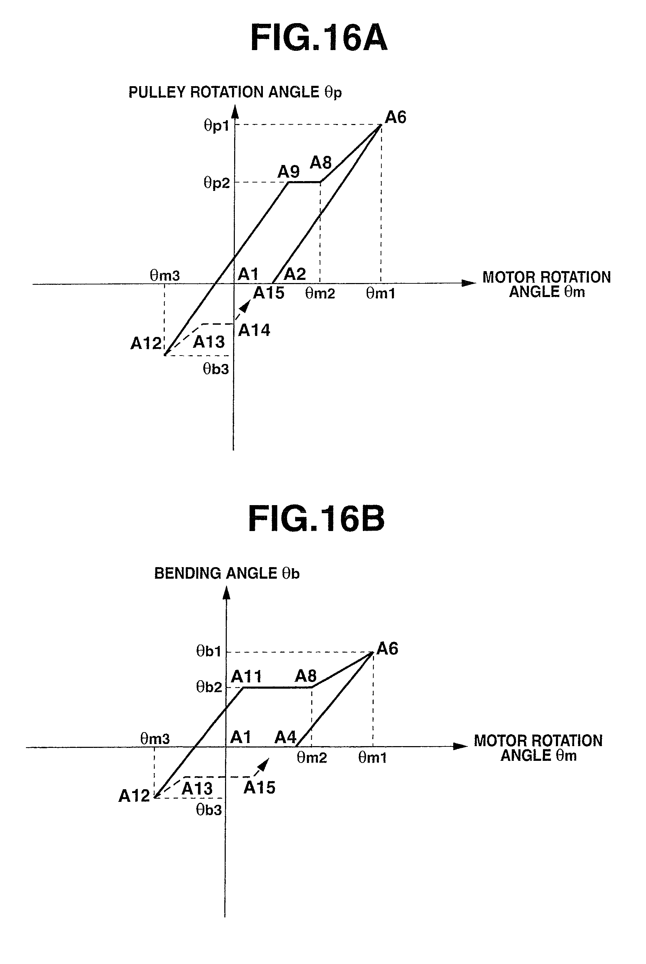

Further, in correspondence with Fig. 15, Fig. 16A and Fig. 16B illustrate that the relationship between characteristics of the motor rotation angle θm and the pulley rotation angle θp, and between the bending angle θb of the bending portion 110 and the motor rotation angle θm each have a characteristic with hysteresis.

The state denoted by reference symbol A1 is a state in which the two engagement surfaces of the motor 109 and the pulley 113 are not engaged by means of the connecting portion 112 (backlashed engagement), and represents a case of a neutral state in which there is slackness in the wires 111a and 111b.

In the state denoted by reference symbol A1, when the motor 109 is rotated in the direction indicated by an arrow that is shown in Fig. 15, the motor rotation angle θm increases from 0, and in the state denoted by reference symbol A2, when a state is entered in which the motor 109 and the pulley 113 have engaged by means of the connecting portion 112, together with rotation of the motor 109 as denoted by reference symbols A2 and A3, the pulley 113 also rotates and the pulley rotation angle θp increases from 0.

In other words, in the state denoted by reference symbols A1 to A2, the pulley rotation angle θp does not change even though the motor 109 rotates and the motor rotation angle θm thereof changes. The relationship characteristic in this case is as shown in Fig. 16A.

In the state denoted by reference symbol A2, since there is slackness in the wire 111a in the pulling direction, in the state denoted by reference symbol A3 in which the motor 109 and the pulley 113 have been rotated further, the bending angle θb of the bending portion 110 does not change from the angle 0 that is the neutral state.

Further, this state continues until the state denoted by reference symbol A4, that is, a state in which the slackness of the wire 111a is removed or cleared. When the motor 109 and pulley 113 rotate to pass the state denoted by reference symbol A4 and reach the state denoted by reference symbol A5, the bending portion 110 bends to the side of the pulled wire 111a.

By making a comparison regarding whether or not the absolute value of the torque value T is greater than or equal to the torque threshold value Tth, the judgment portion 125 judges whether or not the state is a driving force contributing state or torque contributing state in which torque produced by the motor 109 contributes to driving to bend the bending portion 110.

As shown in the lowermost section in Fig. 15, |T|<Tth in the vicinity of the states denoted by reference symbols A1 to A4, and the judgment portion 125 judges that the bending state is not a driving force contributing state.

In contrast, |T|≥Tth in the state denoted by reference symbol A5 that is beyond the state denoted by reference symbol A4. Therefore the judgment portion 125 judges that the bending state is a driving force contributing state. Further, by means of the judgment result of the judgment portion 125, the discrimination portion 127 discriminates whether or not the bending state is the first bending state as the driving force contributing state that contributes to driving to bend the bending portion 110. In the state denoted by reference symbol A5, the discrimination portion 127 discriminates the bending state as being the first bending state.

When the motor 109 is rotated to pass the state denoted by reference symbol A5 and reach a state of a predetermined motor rotation angle θm1 denoted by reference symbol A6, the pulley 113 rotates in conjunction with the rotation of the motor 109, and the bending portion 110 also bends so that a state with a pulley rotation angle θp1 and a bending angle θb1 is entered. The relationship between the motor rotation angle θm and the pulley rotation angle θp in this case is as shown in Fig. 16A, and the relationship between the motor rotation angle θm and the bending angle θb is as shown in Fig. 16B.

After reaching the predetermined motor rotation angle θm1 in this manner, if the motor 109 is rotated in the opposite direction to attempt to bend the bending portion 110 in the opposite direction, even in a state in which a driving signal is not actually applied (supplied) so as to rotate the motor 109, as shown in the bending state denoted by reference symbol A7, the bending portion 110 bends in the direction of the neutral state position by means of the restoring force of an elastic member such as an outer sheath member forming the bending portion 110. Note that, as shown in the states denoted by reference symbol A6 to reference symbol A7 and reference symbol A8, when the bending portion 110 bends in the opposite direction under the restoring force, some of the slackness of the wire 111b is reduced by rotation of the pulley 113.

The restoring force differs in accordance with the size of the bending angle θb1 and a material of the outer sheath member functioning as an elastic member and the like. Particularly, after the bending portion 110 has been bent by a large amount, the restoring force acts with a large force when bending the bending portion 110 in the opposite direction.

When rotating the motor 109 in the opposite direction in this manner (direction that decreases the bending angle 8b), in a state in which the restoring force is acting, a load with respect to the motor 109 becomes less than in a state without a load (A1-A2) or in a state where slackness of the wire is taken up so as to remove the slackness (A2-A4) (these states are referred to collectively as a "state equivalent to no-load").

Therefore, when a torque value in this state that is less than a torque value in a state equivalent to no-load can be detected, this bending state (that is, the second bending state) can also be discriminated based on the detection result.

When the restoring force acts, as shown in the state denoted by reference symbol A8, as a result of the restoring force and frictional forces that act on the wire 111a in resistance to the restoring force and the like, the bending portion 110 bends as far as a specific bending state position as a specific bending state in which the restoring force that bends the bending portion 110 is substantially 0, and the bending portion 110 maintains the state of that bending angle θb2.

In the bending angle θb2 state, since the motor 109 and the pulley 113 are not in an engagement state in which the motor 109 causes the pulley 113 to rotate in the opposite direction, until reaching the state denoted by reference symbol A9 as an engagement state in which the motor 109 and the pulley 113 re-engage from the state denoted by reference symbol A8, even if the motor 109 rotates in the opposite direction, the pulley 113 does not rotate and maintains a fixed pulley rotation angle θp2 value that does not change.

Therefore, it is possible to simply detect the specific bending state position by monitoring a change amount of the motor rotation angle θm and a change amount of the pulley rotation angle θp and, in a state in which the motor rotation angle θm is changing, detecting a state in which the change amount of the pulley rotation angle θp is a change from a negative value to 0 by detecting a case where the threshold value 162b that is close to 0 and the change amount (or absolute value thereof) of the pulley rotation angle θp are compared and the change amount (or absolute value thereof) of the pulley rotation angle θp is less than or equal to the threshold value 162b.

The specific bending state position is a boundary position between the second bending state and the third bending state (number 3-1 bending state). In the present embodiment, a result of this detection is utilized for control of driving for bending.

When the motor 109 is rotated further to pass the state denoted by reference symbol A9, the pulley 113 also rotates together with rotation of the motor 109. However, in this state, since there is slackness in the wire 111 b, until the slackness is removed, that is, until reaching the state denoted by reference symbol A11, the bending portion 110 does not change from the bending angle θb2 in the state denoted by reference symbol A8.

Further, if the motor 109 is rotated to pass through the state denoted by reference symbol A11, together with rotation of the pulley 113 that accompanies rotation of the motor 109, the bending angle θb of the bending portion 110 also changes. The bending portion 110 is bent as far as an appropriate bending angle θb3 in the opposite direction, as denoted by reference symbol A12. Note that, in Fig. 16A and Fig. 16B, the motor rotation angle corresponding to the bending angle θb3 in the state denoted by reference symbol A12 is denoted by θm3, and the pulley rotation angle corresponding thereto is denoted by θp3.

As shown in Fig. 16B, when the bending portion 110 bends in the opposite direction from the bending angle θb3, since in general the absolute values of the bending angles θb1 and θb3 are different, the size of a restoring force differs according to the size of the absolute value of the bending angle θb3. Consequently, as shown by the dotted lines in Fig. 16A and Fig. 16B, hysteresis characteristics (that do not close) as denoted by reference symbols A13, A14 and A15 are exhibited in accordance with the restoring force in the relevant case.

However, in a case with such a hysteresis characteristic also, by identifying a specific bending state that is generated at a specific bending state position or the like, the respective states of the bending angle θb of the bending portion 110, the motor rotation angle θm and the pulley rotation angle θp can be associated and ascertained.

For example, in Fig. 16A, a state denoted by reference symbols A8 to A9 is a state with a characteristic that the change amount of the pulley rotation angle θp2 does not change even if the motor rotation angle θm changes, and a state denoted by reference symbols A13 to A14 exhibits a similar characteristic.

Consequently, by temporally monitoring this state and storing the relevant information in the storage portion 129, characteristics information that reflects a characteristic thereof can be maintained.

By calculating (detecting) temporal changes in operations of the motor 109 and the pulley 113 and the like in this manner and storing information regarding the changes in the storage portion 129, even if the operating state of the motor rotation angle θm and the pulley rotation angle θp exhibits a characteristic with hysteresis, the positional relationship denoted by reference symbol A14 or the like can be ascertained based on the relationship between the two angles, for example, the relationship between the motor rotation angle θm and the pulley rotation angle θp in the state denoted by reference symbol A13.

Thus, according to the present embodiment, the discrimination portion 127 discriminates each of a third bending state (number 3-1 bending state) denoted by reference symbols A1-A2 and A8-A9 in Fig. 15, a third bending state (number 3-2 bending state) denoted by reference symbols A2-A4 and A9-A11 in Fig. 15, a first bending state denoted by reference symbols A4-A6 and A11-A12 in Fig. 15, and a second bending state denoted by reference symbols A6-A8 in Fig. 15. Hence, even when behavior of the bending angle of the bending portion 110, the motor rotation angle θm and the pulley rotation angle θp exhibits a characteristic with hysteresis, the present embodiment enables accurate ascertainment of the states thereof. Further, the motor control portion 121 switches (changes) driving control of the motor 109 in correspondence with a discrimination result obtained by the discrimination portion 127.

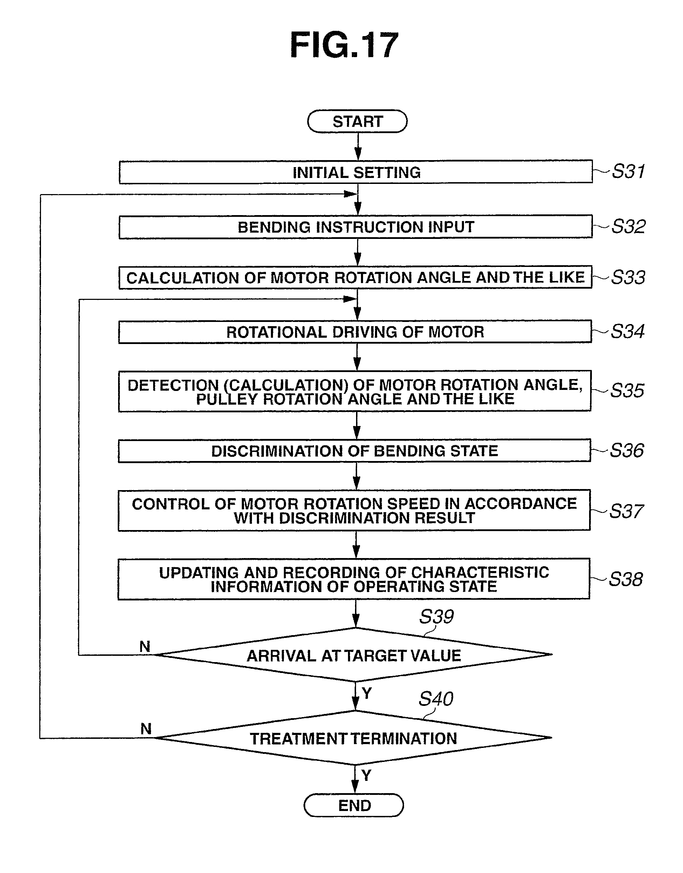

Next, operations according to the present embodiment are described referring to Fig. 17.

When the power of the treatment instrument apparatus 101 is turned on and the control apparatus 104 starts to operate, initial setting processing in step S31 starts.

In step S31, the control apparatus 104 sets a state in which the shaft portion 107 of the treatment instrument 103 is straight, that is, a neutral state in which the bending portion 110 is not bent, and the motor rotation angles θm in the vertical direction and horizontal direction detected by the encoders 114a and 114b (hereinafter, represented by reference symbol "114") of the motors 109a and 109b and the bending angle θb of the bending portion 110 are set to 0. Thereafter, the apparatus is placed on standby for input of an instruction.

In step S32, the operator inputs a bending instruction from the input portion 105. Specifically, the operator operates the joystick 136 to tilt the joystick 136 in the desired direction to bend the bending portion 110 in and at the desired bending angle.

As shown in step S33, in correspondence with the bending direction and bending angle of the inputted instruction, the motor control portion 121 of the control apparatus 104 refers to the characteristics information of the storage portion 129 at that time, and calculates a rotational direction (rotational drive direction) in which to rotate the motors 109a and 109b (hereinafter, represented by reference symbol "109"), a motor rotation angle θsm, a pulley rotation angle θsp, and a torque value (rotational driving force) Ts.

Note that, although the state at this time is the initial state, input of a bending instruction is performed in an operation state which is different from the initial state depending on the control loop in Fig. 17. In such a case, the rotational direction, the motor rotation angle θsm, the pulley rotation angle θsp and the torque value Ts are calculated by referring to information regarding operation characteristics updated prior to the relevant operation state.

The calculated motor rotation angle θsm and torque value Ts serve as instruction values or target values for the motor 109 when driving to bend. Note that, a configuration may also be adopted in which only the motor rotation angle θsm is taken as an instruction value or a target value for the motor 109 when driving to bend.

Next, in step S34, the motor control portion 121 rotationally drives the motor 109 so as to obtain the calculated motor rotation angle 8sm.

At this time, as shown in step S35, the encoder 114 and the potentiometers 115a and 115b (hereinafter, represented by reference symbol "115") and the like each detect the rotation angle of the motor 109 and the pulley 113, respectively. Alternatively, the motor rotation angle calculation portion 122 and the pulley rotation angle calculation portion 123 calculate the motor rotation angle and the pulley rotation angle, respectively. Further, the torque calculation portion 124 calculates the torque value.

That is, the control apparatus 104 detects (calculates) the operating states of the motor 109 and the pulley 113. Further, as shown in step S36, the discrimination portion 127 discriminates the bending state based on the judgment result of the judgment portion 125 or the like.

Subsequently, as shown in step S37, the motor control portion 121 controls the motor rotation speed in accordance with the discrimination result.

In addition, as shown in step S38, the storage portion 129, for example, stores information for the respective operating states of the motor 109 and the pulley 113 in a short fixed cycle, and updates stored characteristics information in accordance with the discrimination result obtained by the discrimination portion 127.

In step S39, the motor control portion 121 discriminates whether or not the motor 109 has been rotated to the target value based on the discrimination result of the discrimination portion 127 or the like into which calculation results of the motor rotation angle calculation portion 122 and the torque calculation portion 124 and the like are inputted.

If the target motor rotation angle θsm has not been reached, the process returns to step S34 to repeat the above described operations. In contrast, if the target value has been reached, in step S40, the control apparatus 104 judges whether or not the treatment is to be ended. If the treatment is not to be ended, the process returns to step S32 to wait for input of the next bending instruction. In contrast, if the treatment is to be ended, the control apparatus 104 ends the processing shown in Fig. 17.

Fig. 18 shows the details of the processing in step S36 and step S37.

As shown in step S41, the discrimination portion 127 makes a discrimination with respect to a first bending state (driving force contributing state) St41a, a second bending state (restoration characteristic state) St41b, and a third bending state St41c. More specifically, as the third bending state St41c, the discrimination portion 127 makes a discrimination with respect to a backlash state St41d as the number 3-1 bending state and a slackened state St41e as the number 3-2 bending state.

If the discrimination result in step S41 is the first bending state, as shown in step S42a, the motor control portion 121 performs control that rotates the motor 109 at a normal rotation speed (referred to as "first rotation speed"). After the processing in step S42a, the process advances to step S38.

If the discrimination result in step S41 is the second bending state, as shown in step S42b, the motor control portion 121 performs control that rotates the motor 109 at a rotation speed corresponding to the restoration characteristic state, specifically, control that causes the motor 109 to rotate at a second rotation speed that is a lower speed than the first rotation speed.

In this state, since the bending portion 110 is already caused to rotate by the restoring force, if rotation is performed at the first rotation speed by the motor 109, the speed will become faster than the speed of normal driving for bending. Hence, by rotating at a lower speed than the first rotation speed, driving for bending is performed in a similar operating state in both a case where the restoring force acts and a case where the restoring force does not act. After the processing in step S42b, the process advances to step S38.

Further, if the discrimination result in step S41 is the number 3-1 bending state (backlash state), as shown in step S42c, the motor control portion 121 performs control that rotates the motor 109 at a third rotation speed corresponding to a backlash state, specifically, control that causes the motor 109 to rotate at a third rotation speed that is a higher speed than the first rotation speed.

Furthermore, if the discrimination result in step S41 is the number 3-2 bending state (slackened state), as shown in step S42d, the motor control portion 121 performs control that rotates the motor 109 at a fourth rotation speed that corresponds to a slackened state. Specifically, the motor control portion 121 performs control to rotate the motor 109 at the fourth rotation speed that is a higher speed than the first rotation speed to wind up the wires 111a and 111b so as to remove slackness therefrom. Note that, the third rotation speed and the fourth rotation speed may be set to the same rotation speed.

In the third bending state, the bending portion 110 corresponds to an unresponsive state in which the bending portion 110 is, in effect, not being bent. Therefore, in the third bending state, by making the rotation speed of the motor 109 a high speed, the motor control portion 121 shortens a time period of the unresponsive state and ensures favorable responsiveness and operability.

By controlling the rotation speed of the motor 109 in accordance with the bending state in this manner, favorable operability can be ensured.

Further, after ending the processing in step S42c, as shown in step S43a, the discrimination portion 127 corrects the current characteristics information stored in the storage portion 129 to characteristics information in which the motor rotation angle and the pulley rotation angle at the bending state position at the time that step S42c ends (when the backlash state is eliminated) are associated, and stores the corrected characteristics information in step S38.

Further, after the processing in step S42d ends, as shown in step S43b, the discrimination portion 127 corrects the current characteristics information stored in the storage portion 129 to characteristics information in which the motor rotation angle, the pulley rotation angle, and the bending angle at the bending state position at the time that step S42d ends (when the slackened state is eliminated) are associated, and stores the corrected characteristics information in step S38.

By performing the above described control processing, the present embodiment detects the occurrence of an unresponsive state such as slackness of the wires 111a and 111b, and controls to update characteristics information in accordance with the slackness and to promptly eliminate the unresponsive state.

Therefore, according to the present embodiment, when using wires in which slackness exists, a bending state that is a case where an unresponsive state occurs in which the bending portion 110 will not be bent can be accurately detected without lowering the responsiveness, and operability when driving to bend the bending portion 110 can be improved.

Furthermore, in the case of driving for bending that exhibits a hysteresis characteristic due to slackness of a wire also, since a configuration is adopted so as to perform a correction with respect to the slackness, a decrease in the accuracy of the control system when driving to bend can be prevented. Note that, to perform rotational control of the motors more simply, a configuration may be adopted in which rotational control is performed taking the above described number 3-1 bending state and number 3-2 bending state collectively as a third bending state.

Note that, although the above embodiment has been described with respect to a case in which the treatment instrument 103 is inserted through a channel of the endoscope 102 and used, the present invention can also be applied to a case where the treatment instrument 103 is used without being inserted through the channel.

The present application is filed claiming the priority of Japanese Patent Applications No. 2011-107423 filed in Japan on May 12, 2011 and No. 2011-107424 filed in Japan on May 12, 2011, and the above described disclosure is incorporated by reference in the present description, claims and drawings.

## Claims

1. A medical control apparatus, comprising:
an insertion portion that is provided in a medical apparatus, and that comprises a bending portion that is formed using an elastic member having flexibility on a distal end side;
a drive portion that generates a driving force for subjecting the bending portion to a bending operation;
a wire that is inserted through an inside of the insertion portion, and that is provided with slackness and connected to the bending portion;
a pulling portion that pulls the wire;
a connecting portion that connects the drive portion and the pulling portion so as to have a positional relationship that transmits the driving force between the drive portion and the pulling portion and a positional relationship that cannot transmit the driving force between the drive portion and the pulling portion;
a driving amount detection portion that detects a driving amount of the drive portion as a drive portion driving amount;
a pulling portion driving amount detection portion that detects a pulling portion driving amount that is pulled by the pulling portion;
a comparison portion that compares a change amount of the drive portion driving amount and a change amount of the pulling portion driving amount; and
an identification portion that, based on a comparison result obtained by the comparison portion, identifies a boundary between a range in which contribution to bending of the bending portion is possible by a restoring force of the elastic member forming the bending portion and a range in which the contribution to bending of the bending portion is not possible due to occurrence of the slackness of the wire.

2. The medical control apparatus according to claim 1,
wherein the connecting portion has a positional relationship that transmits the driving force by contact between an engagement surface of a convex portion and an engagement surface of a concave portion, and also has a positional relationship in which a backlash portion at which both engagement surfaces do not contact against each other cannot transmit the driving force.

3. The medical control apparatus according to claim 2,
wherein, as the boundary, the identification portion identifies a specific bending state position at which the bending portion maintains a specific bending state without the drive portion applying a driving amount to the bending portion during a period until the connecting portion is set in a positional relationship that transmits the driving force from a positional relationship in which the driving force is not transmitted after a bending action caused by the restoring force of the elastic member ends.

4. The medical control apparatus according to claim 3,
wherein, in a state in which a comparison result of the comparison portion is that there is a change amount of the drive portion driving amount, the identification portion identifies a state in which a comparison result of the comparison portion is that a change amount of the pulling portion driving amount is approximately 0 as the specific bending state position.

5. The medical control apparatus according to claim 4, wherein:
the drive portion is configured by a motor that generates a torque as the driving force; and
the pulling portion is configured by a pulley around which a rear end of a wire having a distal end connected to the bending portion is wound and rotatably connected, and that is connected to the motor through the connecting portion.

6. The medical control apparatus according to claim 4,
wherein the connecting portion is formed by one of the convex portion and the concave portion that is connected to a rotation shaft of the motor, and the other of the convex portion portion and the concave portion that is provided in the vicinity of a rotation shaft of the pulley and engages with the one of the convex portion and the concave portion.

7. The medical control apparatus according to claim 3,
wherein the comparison portion determines a difference value between a change amount of the drive portion driving amount and a change amount of the pulling portion driving amount, and performs a comparison with respect to the difference value.

8. The medical control apparatus according to claim 7,
wherein the identification portion identifies the boundary as a case where an absolute value of the difference value that is detected by the comparison portion is less than a predetermined value.

9. The medical control apparatus according to claim 7, further comprising:
a control portion that changes control that pulls and drives the wire by means of the drive portion, based on the boundary that is identified by the identification portion.

10. A medical control apparatus, comprising:
an insertion portion having a bending portion on a distal end side;
a drive portion that generates a driving force for subjecting the bending portion to a bending operation;
a wire that extends from the bending portion and can be pulled by means of the drive portion, and that is connected with slackness to the bending portion;
a judgment portion that judges whether or not a driving force of the drive portion is in a driving force contributing state that contributes to driving to bend the bending portion;
a driving force detection portion that detects a driving force generated at the drive portion;
a position identification portion that detects a specific bending state position at which the bending portion maintains a specific bending state without a driving force that drives to bend the bending portion being applied by the drive portion; and
a discrimination portion that discriminates, as bending states of the bending portion, a first bending state that is the driving force contributing state, a second bending state in which a bending state of the bending portion is changed as far as the specific bending state position immediately after the state is changed from the driving force contributing state to a bending state that is not the driving force contributing state by the drive portion, and a third bending state in which a driving force generated by the drive portion is not detected as a value that is greater than or equal to a predetermined value in a bending state that is not the driving force contributing state excluding the second bending state.

11. The medical control apparatus according to claim 10, further comprising:
a control portion that changes a driving speed at which the drive portion drives, in accordance with a discrimination result of the discrimination portion.

12. The medical control apparatus according to claim 10, further comprising:
a control portion that, in accordance with a discrimination result of the discrimination portion, performs control that pulls the wire at a first driving speed by means of the drive portion in the first bending state, pulls the wire at a second driving speed that is less than the first driving speed by means of the drive portion in the second bending state, and winds up the wire at a third driving speed that is greater than the first driving speed by means of the drive portion in the third bending state.

13. The medical control apparatus according to claim 10, further comprising:
a pulling portion on which a rear end of the wire having a distal end attached to the bending portion is suspended, and which is connected to the drive portion and used for pulling the wire; and
a storage portion that stores information of a driving amount of the drive portion, information of a bending amount of the bending portion that is driven to bend via the pulling portion and the wire by the drive portion, and information of a pulling portion driving amount of the pulling portion as associated characteristics information.

14. The medical control apparatus according to claim 13, wherein:
the drive portion is configured by a motor that generates a torque that is a rotational driving force as the driving force;
the pulling portion is configured by a pulley over which a rear end of a wire having a distal end connected to the bending portion is wound and rotatably suspended; and
the motor and the pulley are rotatably connected by a connecting portion that has a positional relationship that transmits the torque and a positional relationship that cannot transmit the torque.

15. The medical control apparatus according to claim 12,
wherein the position identification portion comprises a pulling portion on which the wire is suspended and which pulls the wire, a connecting portion that connects the drive portion and the pulling portion so as to have a positional relationship that transmits the driving force between the drive portion and the pulling portion and a positional relationship that cannot transmit the driving force between the drive portion and the pulling portion, a driving amount detection portion that detects a driving amount of the drive portion as a drive portion driving amount, a pulling portion driving amount detection portion that detects a pulling portion driving amount that is pulled by the pulling portion, and a comparison portion that performs a comparison with respect to a change amount of the pulling portion driving amount in a case of a change amount in which the drive portion driving amount decreases;
and wherein the comparison portion detects a case of a change amount that substantially stops of the pulling portion driving amount that is a change amount that is less than or equal to a threshold value, as the specific bending state position.

16. The medical control apparatus according to claim 14, wherein:
the position identification portion comprises the connecting portion, an encoder that detects a motor rotational driving amount of the motor as the drive portion driving amount, a potentiometer that detects a pulley rotational driving amount that is pulled by the pulley as the pulling portion driving amount, and a comparison portion that performs a comparison with respect to a change amount of the pulley rotational driving amount in a case of a change amount in which the motor rotational driving amount decreases;
and wherein the comparison portion detects a case where a change amount of the pulley rotational driving amount is approximately 0 that is a change amount that is less than or equal to a threshold value, as the specific bending state position.

17. The medical control apparatus according to claim 14,
wherein the connecting portion has a positional relationship which transmits the driving force by contact between an engagement surface of a convex portion and an engagement surface of a concave portion, and also has a positional relationship of a backlash state in which a backlash portion at which both engagement surfaces do not contact against each other cannot transmit the driving force.

18. The medical control apparatus according to claim 13,
wherein the judgment portion judges whether or not a state is the driving force contributing state based on whether or not an absolute value of a driving force of the drive portion is equal to or greater than a predetermined threshold value.

19. The medical control apparatus according to claim 14,
wherein the judgment portion judges whether or not a state is the driving force contributing state based on whether or not an absolute value of a torque of the motor is equal to or greater than a predetermined threshold value.

20. The medical control apparatus according to claim 14, wherein:
the bending portion is formed using an elastic member that has flexibility and has an elastic force; and
based on a change amount of a pulley rotational driving amount of the pulley, the discrimination portion discriminates a second bending state in which a bending state of the bending portion is changed by a restoring force of the elastic member forming the bending portion immediately after a driving force contributing state is changed to a bending state that is not a driving force contributing state by the motor comprising the drive portion in accordance with a judgment result obtained by the judgment portion

21. The medical control apparatus according to claim 17,
wherein the discrimination portion further discriminates, with respect to the third bending state, the backlash state in a case where a change amount of a pulley rotational driving amount of the pulley is approximately 0 with respect to a change of a rotational driving amount of the motor, and a slackened state in which there is slackness in the wire in a case other than the backlash state in the third bending state.

22. The medical control apparatus according to claim 13, further comprising:
a control portion that, in accordance with a discrimination result of the discrimination portion, performs control to pull the wire at a first driving speed by means of the drive portion in a case of the first bending state, to pull the wire at a second driving speed that is less than the first driving speed by means of the drive portion in a case of the second bending state, to pull the wire at a third driving speed that is greater than the first driving speed in a case of the connecting portion backlash state, and in a case of the slackened state, to pull the wire so as to wind up the wire at a fourth driving speed that is greater than the first driving speed and correct the characteristics information in accordance with an amount of the wire that is wound up.
